(19) Europäisches Patentamt
European Patent Office
Office européen des brevets

(11) EP 4 167 940 B1

(12) EUROPÄISCHE PATENTSCHRIFT

(45) Veröffentlichungstag und Bekanntmachung des Hinweises auf die Patenterteilung:
**09.04.2025 Patentblatt 2025/15**

(21) Anmeldenummer: **21723233.9**

(22) Anmeldetag: **03.05.2021**

(51) Internationale Patentklassifikation (IPC):
***A61K 8/39** (2006.01)* ***A61K 8/58** (2006.01)*
***A61K 8/898** (2006.01)* ***A61Q 5/06** (2006.01)*
***A61Q 5/10** (2006.01)*

(52) Gemeinsame Patentklassifikation (CPC):
**A61K 8/39; A61K 8/585; A61K 8/898; A61Q 5/065; A61Q 5/10**

(86) Internationale Anmeldenummer:
**PCT/EP2021/061543**

(87) Internationale Veröffentlichungsnummer:
**WO 2021/254685 (23.12.2021 Gazette 2021/51)**

# (54) MITTEL ZUM FÄRBEN VON KERATINISCHEN FASERN ENTHALTEND BESTIMMTE NICHTIONISCHE EMULGATOREN, PIGMENTE UND AMINOSILIKONE

AGENT FOR DYEING KERATINOUS FIBERS CONTAINING CERTAIN NONIONIC EMULSIFIERS, PIGMENTS AND AMINOSILICONES

AGENT DE TEINTURE DES FIBRES KÉRATINIQUES CONTENANT CERTAINS ÉMULSIFIANTS NON IONIQUES, DES PIGMENTS ET DES AMINOSILICONES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.06.2020 DE 102020207605**

(43) Veröffentlichungstag der Anmeldung:
**26.04.2023 Patentblatt 2023/17**

(73) Patentinhaber: **Henkel AG & Co. KGaA**
**40589 Düsseldorf (DE)**

(72) Erfinder:
- **KRUCK, Constanze**
  **41515 Grevenbroich (DE)**
- **HILBIG, Sandra**
  **44894 Bochum (DE)**
- **MOCH, Melanie**
  **41542 Dormagen (DE)**
- **KESSLER-BECKER, Daniela**
  **51371 Leverkusen (DE)**

(56) Entgegenhaltungen:
**WO-A1-2018/115059** **WO-A1-2020/088813**
**DE-A1- 102018 222 022**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Gegenstand der vorliegenden Anmeldung ist ein Mittel zum Färben von keratinischem Material, insbesondere menschlichen Haaren, welches mindestens einen nichtionischen Emulgator mit einem HLB-Wert von 4,0 bis 12,0 (a1), mindestens ein Pigment (a2) und mindestens ein aminofunktionalisiertes Silikonpolymer (a3) enthält.

Ein weiterer Gegenstand dieser Anmeldung ist ein Verfahren zum Färben von keratinischem Material, insbesondere menschlichen Haaren, wobei zunächst ein anwendungsbereites Färbemittel durch Vermischen von verschiedenen Mitteln hergestellt wird, wobei diese Mittel mindestens einen nichtionischen Emulator mit einem HLB-Wert von 4,0 bis 12,0 (a1), ein Pigment (a2) und ein aminofunktionalisiertes Silikonpolymer (a3) enthalten. Diese anwendungsbereiten Mittel werden auf die Keratinfasern appliziert, einwirken gelassen und wieder ausgespült.

Ein weiterer Gegenstände dieser Anmeldung sind Mehrkomponenten-Verpackungseinheiten, welche getrennt konfektioniert in verschiedenen Containern die Mittel (I), (II) sowie gegebenenfalls (III) umfassen, wobei diese Mittel nichtionischen Emulator mit einem HLB-Wert von 4,0 bis 12,0 (a1), Pigment (a2) und aminofunktionalisiertes Silikonpolymer (a3) enthalten.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbemitteln und Färbeverfahren. In der letzten Zeit besonders im Fokus stehen Färbesysteme, die auf Pigmenten basieren. Siehe z.B. DE 10 2018 222022 A1 (HENKEL AG & CO KGAA [DE]) 18. Juni 2020 (2020-06-18).

Es war die Aufgabe der vorliegenden Erfindung, ein Färbmittel bereitzustellen, das es ermöglicht, Pigmente in extrem dauerhafter Weise auf den Haaren zu fixieren. Bei Anwendung des Mittels in einem Färbeverfahren sollten besonders intensive Färbeergebnisse erzielt werden. Weiterhin sollten auch Färbungen mit einer guten Waschechtheit, einem guten Egalisiervermögen und einem besonders gleichmäßigen Farbresultat erzielt werden.

Überraschenderweise hat sich nun herausgestellt, dass die vorgenannte Aufgabe hervorragend gelöst werden kann, wenn keratinisches Material, insbesondere menschliche Haare, mit einem Mittel gefärbt werden, welches mindestens einen nichtionischen Emulgator (a1) mit einem HLB-Wert im Bereich von 4,0 bis 12,0, mindestens ein Pigment (a2) und mindestens ein aminofunktionalisiertes Silikonpolymer (a3) enthält.

Ein erster Gegenstand der vorliegenden Erfindung ist ein Mittel zum Färben von keratinischem Material, insbesondere menschlichen Haaren, wie in den Ansprüchen definiert, enthaltend

(a1) mindestens einen nichtionischen Emulgator mit einem HLB-Wert von 4,0 bis 12,0,
(a2) mindestens ein Pigment, und
(a3) mindestens ein aminofunktionalisiertes Silikonpolymer.

Im Rahmen der zu dieser Erfindung führenden Arbeiten konnte herausgefunden werden, dass ganz besonders intensive Farbergebnisse erhalten werden konnten, wenn das oder die Pigmente (a2) in Abmischung mit mindestens einem nichtionischen Emulgator (a1), dessen HLB-Wert in einem bestimmten Bereich liegt, und mindestens einem Aminosilikon (a3) auf das Kertinmateial, insbesondere das menschliche Haar, appliziert wurden.

keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.
Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Mittel zur Färbung

Der Begriff "Mittel zur Färbung" wird im Rahmen dieser Erfindung für eine durch Einsatz von Pigmenten hervorgerufene Farbgebung des Keratinmaterials, insbesondere des Haares, verwendet. Bei dieser Färbung lagern sich die Pigmente als farbgebende Verbindungen in einem besonders homogenen und gleichmäßigen Film an der Oberfläche des Keratinmaterials ab.
Das Färbemittel stellt erfindungsgemäß ein anwendungsbereites Mittel dar. Dieses anwendungsbereite Mittel kann beispielsweise in einen Container abgefüllt und in dieser Form ohne weitere Verdünungs-, Mischungs- oder andere Verfahrens-Schritte auf das Keratinmaterial appliziert werden. Aus Gründen der Lagerstabilität hat sich jedoch als ganz besonders bevorzugt herausgestellt, wenn das anwendungsbereite kosmetische Mittel vom Friseur oder Anwender erst kurz vor der Anwendung hergestellt wird. Zur Herstellung des anwendungsbereiten Mittels kann beispielsweise das Gemisch oder die Vordispersion aus nichtionischem Emulgator (a1) und Pigment (a2) mit einem oder mehreren weiteren Mitteln erfolgen, wobei eines dieser weiteren Mittel mindestens ein aminofunktionalisiertes Silikonpolymer (a3) enthält. Genauso ist jedoch auch denkbar, dass das anwendungsbereite Mittel durch Abmischung von mindestens drei verschiedenen Mitteln hergestellt wird, wobei eines dieser Mittel mindestens einen nichtionischen Emulgator (a1), ein weiteres Mittel mindestens ein Pigment (a2) und noch ein weiteres Mittel mindestens aminofunktionalisiertes Silikonpolymer (a3) beinhaltet. Das Vermischen der Mittel kann zum Beispiel durch Verschütteln erfolgen und gewährleistet auf diese Weise eine ganz besonders gleichmäßige Verteilung der dispergierten Pigmente.
In anderen Worten ist ein erster Gegenstand der vorliegenden Anmeldung ein Mittel, bevorzugt ein anwendungsbereites Mittel, zum Färben von keratinischem Material, insbesondere menschlichen Haaren, enthaltend in einem kosmetischen Träger

(a1) mindestens einen nichtionischen Emulgator mit einem HLB-Wert von 4,0 bis 12,0,
(a2) mindestens ein Pigment, und
(a3) mindestens ein aminofunktionalisiertes Silikonpolymer.

nichtionische Emulgatoren mit einem HLB-Wert von 4,0 bis 12,0 (a1)

Als ersten wesentlichen Inhaltsstoff enthalten die erfindungsgemäßen Mittel mindestens einen nichtionischen Emulgator mit einem HLB-Wert von 4,0 bis 12,0 (a1). Nichtionisch im Sinne der Erfindung bedeutet dabei, dass der Emulgator in wässriger Lösung keine Ionen bildet.
Der Begriff des HLB-Wertes ist den Fachmann gut bekannt und bezeichnet das hydrophil-lipophile Gleichgewicht einesTensids. Der HLB-Wert ist ein von Griffin (1950) eingeführtes Maß für die Wasser- bzw. Öllöslichkeit von Verbindungen, wobei Verbindungen mit niedrigen HLB-Werten hydrophobere Eigenschaften besitzen als Verbindungen mit hohen HLB-Werten.
Für die Definition des HLB-Wertes wird auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.
Unter einem HLB-Wert im Sinne der vorliegenden Anmeldung wird ganz besonders bevorzugt der HLB-Wert nach Griffin verstanden, dessen Berechnung in der Publikation J. Soc. Cosm. Chem. 1954 (Band 5), Seiten 249-256 beschrieben wird.
Die HLB-Werte für nichtionische Tenside können folgendermaßen berechnet werden

$$\mathrm{HLB} = 20 \times \left(1 - \frac{\mathrm{M_l}}{\mathrm{M}}\right)$$

$M_l$ ist die Molmasse des lipophilen Anteils eines Moleküls, und

M ist die Molmasse des gesamten Moleküls.

Bei einem ethoxylierten Fettalkohol entspricht der hydrophile Teil den an der Fettkette berfindlichen Oxyethylen-Einheiten (d.h. den Ethoxy-Einheiten). Bei einem Ester oder Amid entspricht der hydrophile Part des Emulgators dfinitionsgemäß dem Molekülteil sich, beginnend von der Fettkette an, hinter der Carbonylgruppe befindet.
Ohne auf diese Theorie beschränkt zu sein wird vermutet, dass die durch den HLB-Wert charakterisierbare Polarität des nichtionischen Emulgators seine Fähigkeit zur Dispergierung der Pigmente im erfindungsgemäßen Mittel mitbestimmt.
Es hat sich herausgestellt, dass insbesondere Emulgatoren mit mittlerer Polarität das oder die Pigmente besonders gut im Mittel zu dispergieren vermögen und auch zu besonders intensiven Farbergebnissen führen. Daher ist es besonders bevorzugt, einen nichtionischen Emulgator im erfindungsgemäßen Mittel einzusetzen, der einen HLB-Wert von 4,5 bis 11,5, bevorzugt von 5,0 bis 10,0,weiter bevorzugt von 5,5 bis 9,5, und besonders bevorzugt von 6,0 bis 9,0 besitzt.
Im Rahmen einer Ausführungsform ist ein besonders bevorzugtes erfindungsgemäßes Mittel dadurch gekennzeichnet, dass es
(a1) mindestens einen nichtionischen Emulgator mit einem HLB-Wert von 4,5 bis 11,5, bevorzugt von 5,0 bis 10,0,weiter bevorzugt von 5,5 bis 9,5, und besonders bevorzugt von 6,0 bis 9,0 enthält.

Als Beispiele für geeignete nichtionische Emulgatoren (a1) können genannt werden

| INCI | Kommerzieller Anbieter | HLB-Wert |
|---|---|---|
| Laureth-2 | Arlypon F (BASF) | 6,5 |
| Laureth-3 | Marlipal 2430 (Sasol) | 8 |
| Laureth-4 | Dehydol LS4 (BASF) | 9,7 |
| Steareth-2 | Brij 72 (Uniqema) | 4.9* |
| Steareth-3 | Isoxal 5 (Vevy) | 6.6* |
| Steareth-4 | Nikkol BS-4 (Nikko) | 7.9* |
| Steareth-5 | Jeecol SA-5 (Jeen) | 9.0* |
| Steareth-6 | Emalex 606 (Nihon Emulsion) | 9.9* |
| Ceteth-2 | Brij 52 (Uniqema) | 5.3* |
| Ceteth-3 | Emalex 103 (Nihon Emulsion) | 7.1* |
| Ceteth-4 | Lipocol C-4 (Lipo) | 8.4* |
| Ceteth-5 | Volpo C5 (Croda) | 9.5* |
| Ceteareth-2 | Volpo CS2 (Croda) | 5.1* |
| Ceteareth-3 | Jeecol CS-3 (Jeen) | 6.8* |
| Ceteareth-4 | Lipocol SC-4 (Lipo) | 8.1* |
| Ceteareth-5 | Volpo CS5 (Croda) | 9.2* |
| Beheneth-5 | Nikkol BB-5 (Nikko) | 8.1* |
| Cocamide MEA | Comperlan 100 (Cognis) | 4.8 |
| Cocamide MIPA | Ninol M-10 (Stepan) | 5.6 |
| Cocamide DEA | Comperlan KD (Cognis) | 7.1 |
| Stearamide DEA | Lipamide S (Lipo) | 5.6 |
| Myristamide DEA | Jeemide MRCA (Jeen) | 6.6 |
| Myristamide MEA | Witcamide MM (Witco) | 4.4 |
| Polyglyceryl-2 distearate | Emalex DSG-2 (Ikeda) | 4.7 |

(fortgesetzt)

| INCI | Kommerzieller Anbieter | HLB-Wert |
|---|---|---|
| Polyglyceryl-3 distearate | Cithrol 2623 (Croda) | 6.2 |
| Polyglyceryl-2 stearate | Nikkol DGMS (Nikko) | 7.6 |
| Polyglyceryl-3 stearate | Radiasurf 7248 (Atofina) | 9.4 |
| Sorbitan distearate | Sorbon S-66 (Toho) | 4.7 |
| Sorbitan palmitate | Span 40 (Uniqema) | 8.1 |
| Sorbitan stearate | Span 60 (Uniqema) | 7.6 |
| Myristyl glucoside | Montanov 14 (SEPPIC) | 9.5 |
| Cetearyl glucoside | Tego Care CG 90 (Degussa) | 8.6 |
| Arachidyl glucoside | Montanov 202 (SEPPIC) | 7.8 |
| * entsprechend HLB-Werten berechnet nach Griffin | | |

In einer weiteren bevorzugten Ausführungsform ist ein erfindungsgemäßes Mittel daher dadurch gekennzeichnet, dass es mindestens einen nichtionischen Emulgator (a1) enthält, der ausgewählt ist aus der Gruppe aus Laureth-2, Laureth-3, Laureth-4, Steareth-2, Steareth-3, Steareth-4, Steareth-5, Steareth-6, Ceteth-2, Ceteth-3, Ceteth-4, Ceteth-5 Ceteareth-2, Ceteareth-3, Ceteareth-4, Ceteareth-5, Beheneth-5, Cocamide MEA, Cocamide MIPA, Cocamide DEA, Stearamide DEA, Myristamide DEA, Myristamide MEA, Polyglyceryl-2 distearate, Polyglyceryl-3 distearate, Polyglyceryl-2 stearate, Polyglyceryl-3 stearate, Sorbitan distearate, Sorbitan palmitate, Sorbitan stearate, Myristyl glucoside, Cetearyl glucoside und Arachidyl glucoside.

Innerhalb dieser Gruppe nochmals ganz besonders bevorzugt sind die nichtionischen Emulgatoren (a1) aus der Gruppe aus Laureth-2, Laureth-3, Laureth-4, Steareth-2, Steareth-3, Steareth-4, Steareth-5, Steareth-6, Ceteth-2, Ceteth-3, Ceteth-4, Ceteth-5 Ceteareth-2, Ceteareth-3, Ceteareth-4, Ceteareth-5 und Beheneth-5.

In einer weiteren ganz besonder bevorzugten Ausführungsform ist ein erfindungsgemäßes Mittel daher dadurch gekennzeichnet, dass es mindestens einen nichtionischen Emulgator (a1) enthält, der ausgewählt ist aus der Gruppe aus Laureth-2, Laureth-3, Laureth-4, Steareth-2, Steareth-3, Steareth-4, Steareth-5, Steareth-6, Ceteth-2, Ceteth-3, Ceteth-4, Ceteth-5 Ceteareth-2, Ceteareth-3, Ceteareth-4, Ceteareth-5 und Beheneth-5.

Die vorgenannten ganz besonders gut geeigneten nichtionischen Emulgatoren (a1) sind ausgewählt aus der Gurppe der ethoxylierten Fettalkohole, wobei insbesondere diejenigen Fettalkohole mit einem niedrigen Ethoxylierungsgrad gute Ergebnisse mit besonders hoher Farbintensität geliefert haben. Entsprechende niedrige ethoxylierte Fettalkohole sind Verbindungen der allgemeinen Formel (E-I),

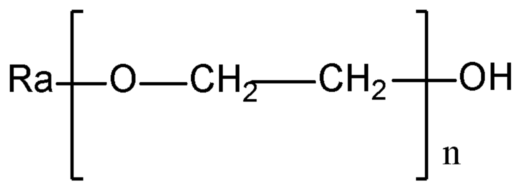

(E-I)

worin

Ra für eine gesättigte oder ungesättigte, unverzweigte oder verzweigte $C_{12}$-$C_{24}$-Alkylgruppe steht und

n für eine ganze Zahl von 1 bis 6, bevorzugt für eine ganze Zahl von 1 bis 5, und besonders bevorzugt für eine ganze Zahl von 2 bis 4 steht.

In einer weiteren bevorzugten Ausführungsform ist ein erfindungsgemäßes Mittel daher dadurch gekennzeichnet, dass es mindestens einen nichtionischen Emulgator (a1) der Formel (E-I) enthält,

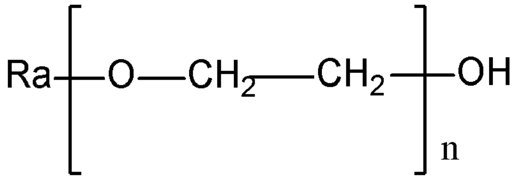

(E-I)

worin

Ra für eine gesättigte oder ungesättigte, unverzweigte oder verzweigte $C_{12}$-$C_{24}$-Alkylgruppe steht und

n für eine ganze Zahl von 1 bis 6, bevorzugt für eine ganze Zahl von 1 bis 5, und besonders bevorzugt für eine ganze Zahl von 2 bis 4 steht.

Der Rest Ra steht für eine gesättigte oder ungesättigte, unverzweigte oder verzweigte $C_{12}$-$C_{24}$-Alkylgruppe. Besonders bevorzugt steht der Rest Ra für eine gesättigte, unverzweigte $C_{12}$-$C_{24}$-Alkylgruppe. Ganz besonders bevorzgt steht der Rest Ra für eine gesättigte, unverzweigte $C_{12}$-$C_{18}$-Alkylgruppe.

Als Beispiele für eine gesättigte, unverzweigte $C_{12}$-$C_{24}$-Alkylgruppe kann beispielsweise eine lineare $C_{12}$-Alkylgruppe, eine lineare $C_{14}$-Alkylgruppe, eine lineare $C_{16}$-Alkylgruppe, eine lineare $C_{18}$-Alkylgruppe, eine lineare $C_{20}$-Alkylgruppe, eine lineare $C_{22}$-Alkylgruppe und eine lineare $C_{24}$-Alkylgruppe genannt werden.

Die Indexzahl n steht für eine ganze Zahl von 1 bis 6, bevorzugt für eine ganze Zahl von 1 bis 5, und besonders bevorzugt für eine ganze Zahl von 2 bis 4.

In einer weiteren ganz bevorzugten Ausführungsform ist ein erfindungsgemäßes Mittel daher dadurch gekennzeichnet, dass es mindestens einen nichtionischen Emulgator (a1) der Formel (E-I) enthält,

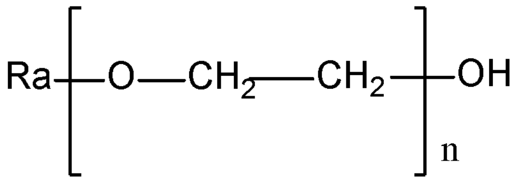

(E-I)

worin

Ra für eine gesättigte, unverzweigte $C_{12}$-$C_{18}$-Alkylgruppe steht und

n für eine ganze Zahl von 1 bis 6, bevorzugt für eine ganze Zahl von 1 bis 5, und besonders bevorzugt für eine ganze Zahl von 2 bis 4 steht.

Ein ganz besonders gut geeigneter nichtionischer Emulgator (a1) ist beispielweise Laureth-2, welcher die CAS-Nummer 68439-50-9 trägt und unter dem Handelsnamen Arlypon F von der Firma BASF kommerziell vertrieben wird. Laureth-2 kann auch unter dem Handelsnamen Oxetal VD 20 von der Firma Zschimmer & Schwarz käuflich erworben werden.

Um eine besonders feine Dispergierung der Pigmente (a2) im erfindungsgemäßen Mittel zu gewährleisten, werden das oder die nichtionischen Emulgatoren (a1) bevorzugt in bestimmten Mengenbereichen eingesetzt. So hat es sich als besonders vorteilhaft herausgestellt, wenn das Mittel - bezogen auf sein Gesamtgewicht - einen oder mehrere nichtionische Emulgatoren (a1), deren HLB-Werte in dem zuvor beschriebenen erfindungsgemäßen Bereich liegen, in einer Gesamtmenge von 0,1 bis 10,0 Gew.-%, bevorzugt von 0,2 bis 8,0 Gew.-%, weiter bevorzugt von 0,4 bis 6,0 Gew.-% und ganz besonders bevorzugt von 0,8 bis 2,5 Gew.-% enthält.

In einer weiteren besonders bevorzugten Ausführungsform ist ein erfindungsgemäßes Mittel dadurch gekennzeichnet, dass es - bezogen auf das Gesamtgewicht des Mittels - einen oder mehrere nichtionische Emulgatoren (a1) in einer Gesamtmenge von 0,1 bis 10,0 Gew.-%, bevorzugt von 0,2 bis 8,0 Gew.-%, weiter bevorzugt von 0,4 bis 6,0 Gew.-% und ganz besonders bevorzugt von 0,8 bis 2,5 Gew.-% enthält.

In einer weiteren besonders bevorzugten Ausführungsform ist ein erfindungsgemäßes Mittel dadurch gekennzeichnet, dass es - bezogen auf das Gesamtgewicht des Mittels - 0,1 bis 10,0 Gew.-%, bevorzugt von 0,2 bis 8,0 Gew.-%, weiter bevorzugt von 0,4 bis 6,0 Gew.-% und ganz besonders bevorzugt von 0,8 bis 2,5 Gew.-% Laurteh-2 enthält.

Pigmente (a2)

Als zweiten wesentlichen Bestandteil enthält das erfindungsgemäße Mittel mindestens ein Pigment (a2). Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Mittel dadurch gekennzeichnet, dass es mindestens eine farbgebende Verbindung (a2) aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Mittel dadurch gekennzeichnet, dass es mindestens ein anorganisches Pigment (a2) enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es mindestens eine farbgebende Verbindung (a2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)

Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch ein oder mehrere farbgebende Verbindungen (a2) aus der Gruppe der organischen Pigmente enthalten
Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Mittel dadurch gekennzeichnet, dass es mindestens ein organisches Pigment (a2) enthält, das bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI

19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem erfindungsgemäßen Mittel ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße $D_{50}$ von 1,0 bis 50 $\mu$m, vorzugsweise von 5,0 bis 45 $\mu$m, bevorzugt von 10 bis 40 $\mu$m, insbesondere von 14 bis 30 $\mu$m, aufweist. Die mittlere Teilchengröße $D_{50}$ kann beispielsweise unter Verwendung von dynamischer Lichtstreuung (DLS) bestimmt werden.

Die Pigmente (a2) stellen den zweiten wesentlichen des erfindungsgemäßen Mittels dar und werden bevorzugt in bestimmten Mengenbereichen im Mittel eingesetzt.

Besonders gute Ergebnisse wurden erhalten, wenn das Mittel - bezogen auf das Gesamtgewicht des Mittels - ein odere mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthielt.

In einer weiteren ganz besonders bevorzugten Ausführungsform ist ein erfindungsgemäßes Mittel dadurch gekennzeichnet, dass das Mittel - bezogen auf das Gesamtgewicht des Mittels - ein odere mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

In einer weiteren ganz besonders bevorzugten Ausführungsform ist ein erfindungsgemäßes Mittel dadurch gekennzeichnet, dass das Mittel - bezogen auf das Gesamtgewicht des Mittels - ein odere mehrere anorganische Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

In einer weiteren ganz besonders bevorzugten Ausführungsform ist ein erfindungsgemäßes Mittel dadurch gekennzeichnet, dass das Mittel - bezogen auf das Gesamtgewicht des Mittels - ein odere mehrere organische Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

<u>aminofunktionalisierte Silikonpolymere (a3)</u>

Als dritten erfindungswesentlichen Inhaltsstoff enthält das erfindungsgemäße Mittel mindestens ein aminofunktionalisiertes Silikonpolymer (a3). Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des Silikonpolymers nicht mehr als $10^7$ g/mol, bevorzugt nicht mehr als $10^6$ g/mol und besonders bevorzugt nicht mehr als $10^5$ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Prinzipiell konnten gute Effekte aminofunktionalisierten Silikonpolymeren (a3) erzielt werden, wenn diese mindestens eine primäre, mindestens eine sekundäre und/oder mindestens eine tertiäre Aminogruppe tragen. Färbungen mit den höchsten Farbintensitäten wurden jedoch beobachtet, wenn ein aminofunktionalisiertes Silikonpolymer (a3) im Mittel eingesetzt wurde, welches mindestens eine sekundäre Aminogruppe enthält.

In einer ganz besonders bevorzugten Ausführungsform ist ein erfindungsgemäßes Mittel dadurch gekennzeichnet, dass es zusätzlich

(a3) mindestens ein aminofunktionalisiertes Silikonpolymer mit mindestens einer sekundären Aminogruppe, enthält.

Die sekundäre Aminogruppe(n) kann bzw. können sich an verschiedenen Positionen des aminofunktionalisierten Silikonpolymers befinden. Ganz besonders gute Effekt wurden gefunden, wenn ein aminofunktionalisiertes Silikonpolymer (a3) eingesetzt wurde, dass mindestens eine, bevorzugt mehrere Struktureinheiten der Formel (Si-Amino) besitzt.

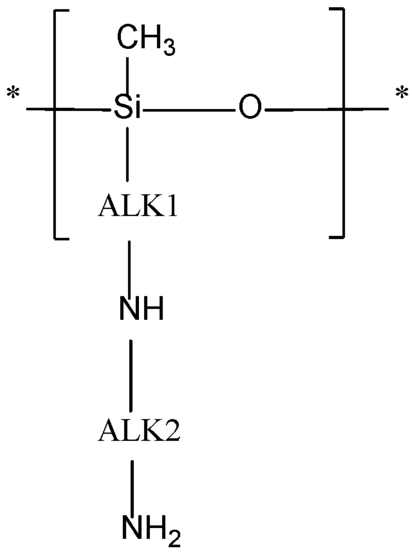

(Si-Amino)

In den Struktureinheiten der Formel (Si-Amino) steht die Kürzel ALK1 und ALK2 unabhängig voneinander für eine lineare oder verzweigte, zweiwertige $C_1$-$C_{20}$-Alkylengruppe.

In einer weiteren ganz besonders bevorzugten Ausführungsform ist ein erfindungsgemäßes Mittel dadurch gekennzeichnet, dass das Mittel mindestens ein aminofunktionalisiertes Silikonpolymer (a3) enthält, das mindestens eine Struktureinheit der Formel (Si-Amino) umfasst,

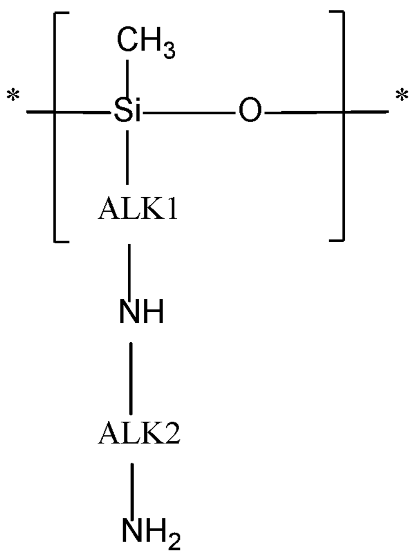

(Si-Amino)

wobei

ALK1 und ALK2 unabhängig voneinander für eine lineare oder verzweigte, zweiwertige $C_1$-$C_{20}$-Alkylengruppe stehen.

Die mit einem Stern (*) gekennzeichneten Position geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch an eine $C_1$-$C_6$-Alkylgruppe gebunden sein.

Eine zweiwertige $C_1$-$C_{20}$-Alkylengruppe kann alternativ auch als eine divalente oder zweibindige $C_1$-$C_{20}$-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige $C_1$-$C_{20}$-Alkylengruppe sind beispielsweise die Methylen-gruppe ($-CH_2-$), die Ethylengruppe ($-CH_2-CH_2-$), die Propylengruppe ($-CH_2-CH_2-CH_2-$) und die Butylengruppe ($-CH_2-CH_2-CH_2-CH_2-$). Die Propylengruppe ($-CH_2-CH_2-CH_2-$) ist besonders bevorzugt. Ab einer Kettenlänge von 3 C-Atomen können zweiwertige Alkylengruppen auch verzweigt sein. Beispiele für verzweigte, zweiwertige $C_3$-$C_{20}$-Alkylengruppen sind ($-CH_2-CH(CH_3)-$) und ($-CH_2-CH(CH_3)-CH_2-$).

In einer weiteren besonders bevorzugten Ausführungsform stellen die Struktureinheiten der Formel (Si-Amino) Wiederholungseinheiten im aminofunktionalisierten Silikonpolymer (a3) dar, so dass das Silikonpolymer mehrer Struktureinheiten der Formel (Si-Amino) umfasst.

Im Folgenden werden besonders gut geeignete aminofunktionalisierte Silikonpolymere (a3) mit mindestens einer sekundären Aminogruppe aufgelistet.

Färbungen mit den allerhöchsten Farbintensitäten konnten erhalten werden, wenn ein Mittel auf dem keratinischen Material appliziert wurde, das mindestens ein aminofunktionalisiertes Silikonpolymer (a3) enthält, das Struktureinheiten der Formel (Si-I) und der Formel (Si-II) umfasst

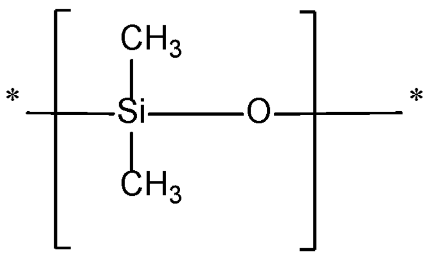

(Si-I)

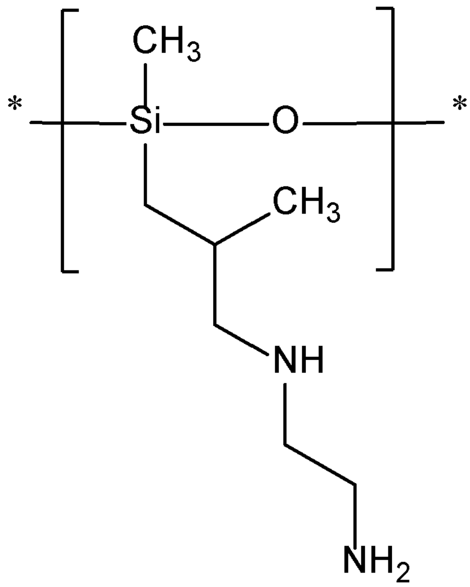

(Si-II).

In einer weiteren explizit ganz besonders bevorzugten Ausführungsform ist ein erfindungsgemäßes Mittel dadurch gekennzeichnet, dass es mindestens ein aminofunktionalisiertes Silikonpolymer (a3) enthält, das Struktureinheiten der Formel (Si-I) und der Formel (Si-II) umfasst

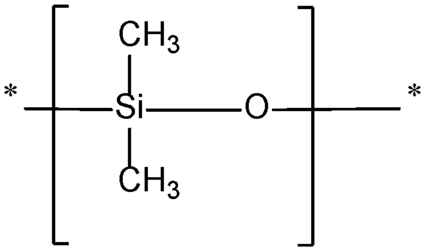

(Si-I)

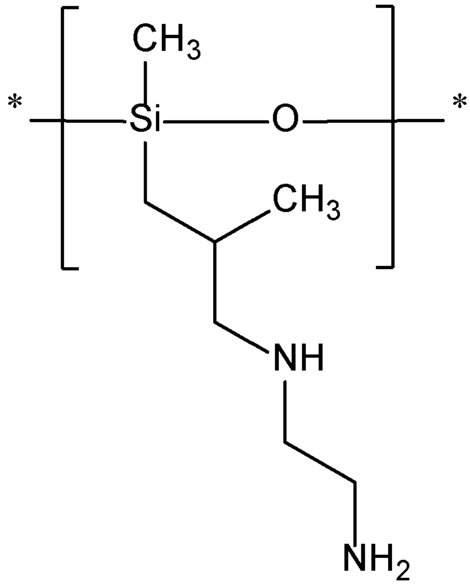

(Si-II).

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt.

Im Rahmen einer weiteren bevorzugten Ausführungsform ist ein erfindungsgemäßes Mittel dadurch gekennzeichnet dass es mindestens ein aminofunktionelles Silikonpolymer (a3) der Formel der Formel (Si-III) enthält,

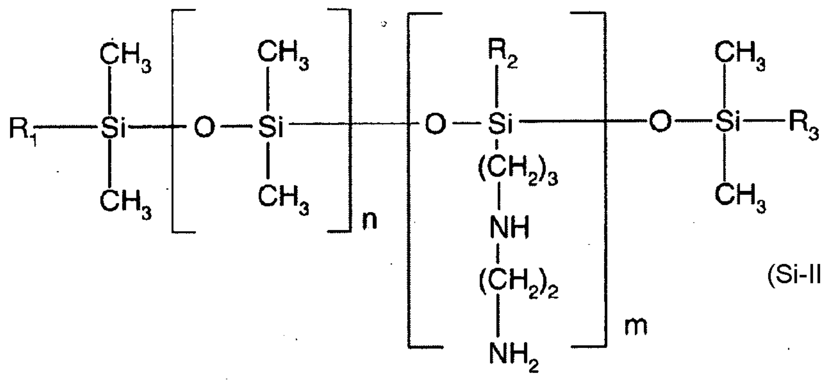

(Si-III)

wobei

- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Mittel sind gekennzeichnet durch ihren Gehalt an mindestens einem aminofunktionellen Silikonpolymer (a3) der Formel der Formel (Si-IV) enthält,

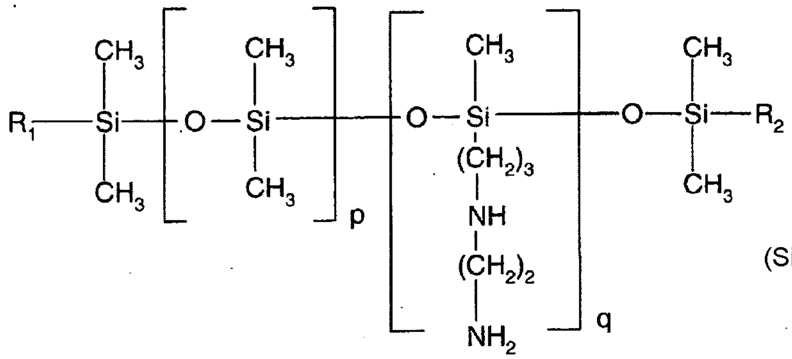

(Si-IV)

in der

- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-$Si(CH_3)_2$-Gruppe an eine -[O-$Si(CH_3)_2$]-Gruppierung gebunden.

Als besonders wirkungsvoll im Hinblick auf die gewünschten Effekte haben sich auch erfindungsgemäße Mittel erwiesen, welche mindestens ein aminofunktionelles Silikonpolymer (a3) der Formel der Formel (Si-V) enthalten

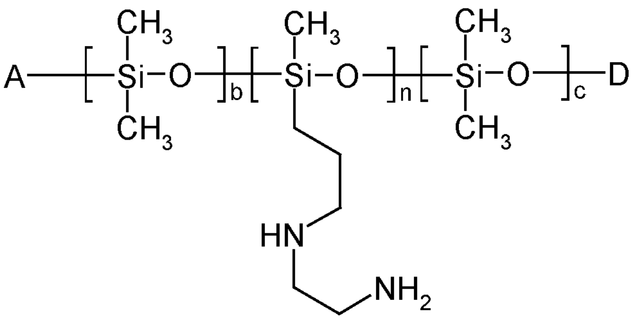

(Si-V),

in der

| | |
|---|---|
| A | für eine Gruppe -OH, -O-$Si(CH_3)_3$,-O-$Si(CH_3)_2OH$ ,-O-$Si(CH_3)_2OCH_3$ steht, |
| D | für eine Gruppe -H, -$Si(CH_3)_3$,-$Si(CH_3)_2OH$, -$Si(CH_3)_2OCH_3$ steht, |
| b, n und c | für ganze Zahlen zwischen 0 und 1000 stehen, mit den Maßgaben |

- n > 0 und b + c > 0
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das Mittel (a) kann weiterhin auch ein oder mehrere verschiedene aminofunktionalisierte Silikonpolymere enthalten, die durch die Formel (Si-VI)

$$M(R_aQ_bSiO_{(4-a-b)/2)x}(R_cSiO_{(4-c)/2)y}M \qquad \text{(Si-VI)}$$

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel $-R^1HZ$ ist, worin $R^1$ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen, $-CH_2CH(CH_3)CH_2-$, Phenylen, Naphthylen, $-CH_2CH_2SCH_2CH_2-$, $-CH_2CH_2OCH_2-$, $-OCH_2CH_2-$, $-OCH_2\ CH_2CH_2-$, $-CH_2CH(CH_3)C(O)OCH_2-$, $-(CH_2)_3\ CC(O)OCH_2CH_2-$, $-C_6H\ _4C_6H_4-$, $-C_6H\ _4CH_2C_6H_4-$; und $-(CH\ _2)_3C(O)SCH_2CH_2-$ ein.

Z ist ein organischer, aminofunktioneller Rest, enthaltend mindestens eine funktionelle Aminogruppe. Eine mögliche Formel für Z ist $NH(CH_2\ )_zNH_2$, worin z 1 oder mehr ist. Eine andere mögliche Formel für Z ist $-NH(CH_2)_z(CH\ _2)_{zz}NH$, worin sowohl z als auch zz unabhängig 1 oder mehr sind, wobei diese Struktur Diamino-Ringstrukturen umfaßt, wie Piperazinyl. Z ist am bevorzugtesten ein $-NHCH_2CH\ _2NH_2$-Rest. Eine andere mögliche Formel für Z ist $-\ N(CH_2)_z(CH_2)_{zz}NX_2$ oder $-NX_2$, worin jedes X von $X_2$ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel $-CH_2CH_2CH_2NHCH_2CH_2NH\ _2$. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der $R_aQ_b\ SiO_{(4-a-b)/2}$-Einheiten zu den $R_cSiO_{(4-c)/2}$-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer weiteren bevorzugten Ausführungsform ist ein erfindungsgemäßes Mittel dadurch gekennzeichnet dass es mindestens ein aminofunktionelles Silikonpolymer der Formel (Si-VII) enthält,

$$R'_aG_{3-a}\text{-}Si(OSiG_2)_n\text{-}(OSiG_bR'_{2-b})_m\text{-}O\text{-}SiG_{3-a}\text{-}R'_a \qquad \text{(Si-VII)},$$

worin bedeutet:

- G ist-H, eine Phenylgruppe, -OH, $-O-CH_3$, $-CH_3$, $-O-CH_2CH_3$, $-CH_2CH_3$, $-O-CH_2CH_2CH_3$,$-CH_2CH_2CH_3$, $-O-CH(CH_3)_2$, $-CH(CH_3)_2$, $-O-CH_2CH_2CH_2CH_3$, $-\ CH_2CH_2CH_2CH_3$, $-O-CH_2CH(CH_3)_2$, $-CH_2CH(CH_3)_2$, $-O-CH(CH_3)CH_2CH_3$, $-\ CH(CH_3)CH_2CH_3$, $-O-C(CH_3)_3$, $-C(CH_3)_3$ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus

  ◦ $-Q-N(R'')-CH_2-CH_2-N(R'')_2$
  ◦ $-Q-N(R'')_2$
  ◦ $-Q-N^+(R'')_3A^-$
  ◦ $-Q-N^+H(R'')_2A^-$
  ◦ $-Q-N'H_2(R'')A^-$
  ◦ $-Q-N(R'')-CH_2-CH_2-N^+R''H_2A^-$,

  wobei jedes Q für eine chemische Bindung, $-CH_2-$, $-CH_2-CH_2-$, $-CH_2CH_2CH_2-$, $-C(CH_3)_2-$, $-CH_2CH_2CH_2CH_2-$, $-CH_2C(CH_3)_2-$, $-CH(CH_3)CH_2CH_2-$ steht,

R" für gleiche oder verschiedene Reste aus der Gruppe -H, -Phenyl, -Benzyl, $-CH_2-CH(CH_3)Ph$, der $C_{1-20}$-Alkylreste, vorzugsweise $-CH_3$, $-CH_2CH_3$, $-CH_2CH_2CH_3$, $-CH(CH_3)_2$, $-CH_2CH_2CH_2H_3$, $-CH_2CH(CH_3)_2$, $-CH(CH_3)CH_2CH_3$, $-C(CH_3)_3$, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Mittel dadurch gekennzeichnet, dass es mindestens ein aminofunktionelles Silikonpolymer (a3) der Formel (Si-VIIa) enthält,

$$(CH_3)_3Si\text{-}[O\text{-}Si(CH_3)_2]_n[OSi(CH_3)]_m\text{-}OSi(CH_3)_3 \qquad \text{(Si-VIIa)},$$

$$|$$

$$CH_2CH(CH_3)CH_2NH(CH_2)_2NH_2$$

worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Mittel dadurch gekennzeichnet, dass es mindestens ein aminofunktionelles Silikonpolymer der Formel (Si-VIIb) enthält

$$R\text{-}[Si(CH_3)_2\text{-}O]_{n1}[Si(R)\text{-}O]_m\text{-}[Si(CH_3)_2]_{n2}\text{-}R \qquad \text{(Si-VIIb)},$$

$$|$$

$$(CH_2)_3NH(CH_2)_2NH_2$$

enthalten, worin R für -OH, $-O-CH_3$ oder eine $-CH_3$-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikonpolymer (a3) enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Mittel geeignet, welche ein spezielles 4-Morpholinomethyl-substituiertes Silikonpolymer (a3) enthielten. Dieses aminofunktionalisierte Silikonpolymer umfasst Struktureinheiten der Formeln (SI-VIII) und der Formel (Si-IX)

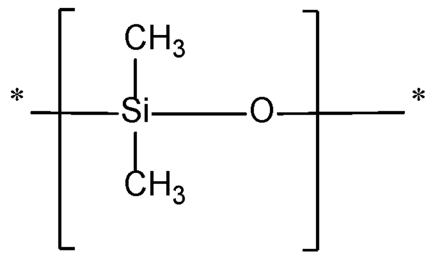

(Si-VIII)

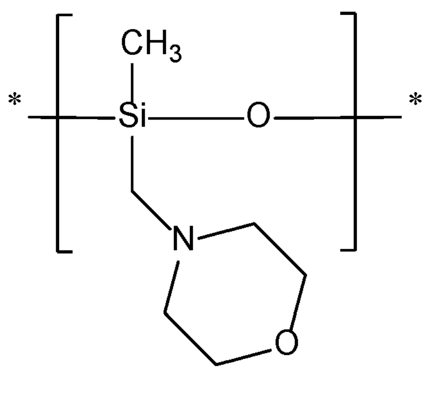

(Si-IX).

Entsprechende 4-Morpholinomethyl-substituiertes Silikonpolymere werden im folgenden beschrieben.

Ein bevorzugtes aminofunktionaliserte Silikonpolymer ist unter dem Namen Amodimethicone/Morpholinomethyl Silsesquioxane Copolymer bekannt und in Form des Rohstoffes Belsil ADM 8301 E von Wacker kommerziell

erhältlich.

Als 4-morpholinomethyl-substituiertes Silikon kann beispielsweise ein Silikon eingesetzt werden, welches Struktureinheiten der Formeln (Si-VIII), (Si-IX) und (Si-X) aufweist

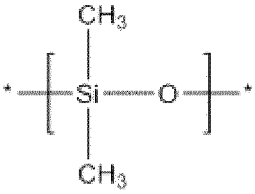
(Si-VIII),
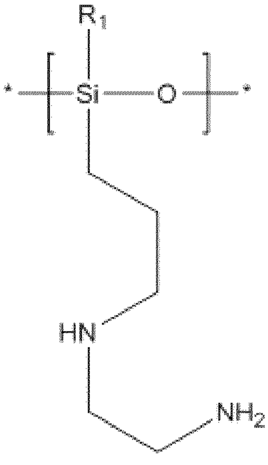
(Si-X)
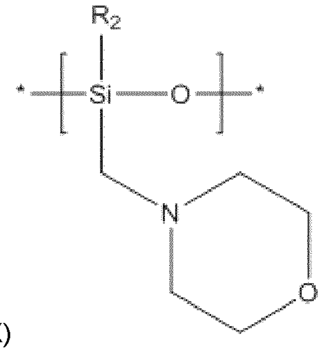
(Si-IX)

in denen

R1 für $-CH_3$, -OH, $-OCH_3$, $-O-CH_2CH_3$, $-O-CH_2CH_2CH_3$, oder $-O-CH(CH_3)_2$ steht;
R2 für $-CH_3$, -OH, oder $-OCH_3$ steht.

Besonders bevorzugte erfindungsgemäße Mittel enthalten mindestens ein 4-morpholinomethylsubstituierten Silikons der Formel (Si-XI)

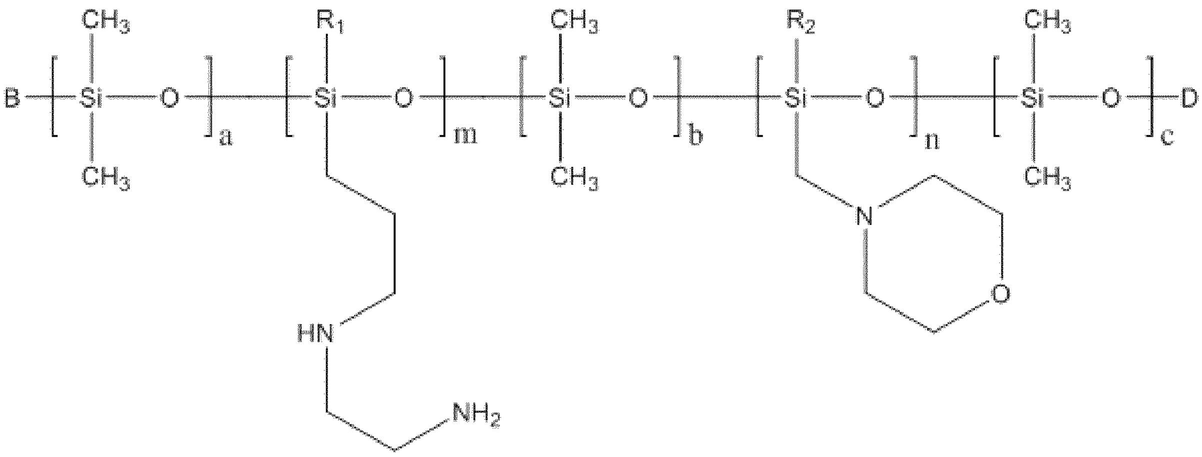

(Si-XI)

in der

R1 für $-CH_3$, -OH, $-OCH_3$, $-O-CH_2CH_3$, $-O-CH_2CH_2CH_3$, oder $-O-CH(CH_3)_2$ steht;
R2 für $-CH_3$, -OH, oder $-OCH_3$ steht.
B für eine Gruppe -OH, $-O-Si(CH_3)_3$,$-O-Si(CH_3)_2OH$ ,$-O-Si(CH_3)_2OCH_3$ steht,
D für eine Gruppe -H, $-Si(CH_3)_3$,$-Si(CH_3)_2OH$, $-Si(CH_3)_2OCH_3$ steht,

a, b und c unabhängig voneinander für ganze Zahlen zwischen 0 und 1000 stehen, mit der Maßgabe a + b + c> 0
m und n unabhängig voneinander für ganze, Zahlen zwischen 1 und 1000 stehen mit den Maßgabe, daß

- mindestens eine der Bedingungen B = -OH bzw. D = -H erfüllt ist,
- die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = $-Si(CH_3)_3$), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

B = $-O-Si(CH_3)_2OH$ und D = $-Si(CH_3)_3$
B = $-O-Si(CH_3)_2OH$ und D = $-Si(CH_3)_2OH$
B = $-O-Si(CH_3)_2OH$ und D = $-Si(CH_3)_2OCH_3$
B = $-O-Si(CH_3)_3$ und D = $-Si(CH_3)_2OH$
B = $-O-Si(CH_3)_2OCH_3$ und D = $-Si(CH_3)_2OH$

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Es hat sich als besonders vorteilhaft herausgestellt, wenn das erfindungsgemäße Mittel das oder die aminofunktionalisierten Silikonpolymere (a3) in bestimmten Mengenbereichen enhält. Besonders gute Ergebnisse konnten erhalten werden, wenn das Mittel - bezogen auf das Gesamtgewicht des Mittels - in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

Im Rahmen einer weiteren besonders bevorzugten Ausführungsform ist ein erfindungsgemäßes Mittel dadurch gekennzeichnet, dass es - bezogen auf das Gesamtgewicht des Mittels - ein oder mehrere aminofunktionalisierte Silikonpolymere (a3) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

weitere optionale Bestandteile im Mittel

Abhängig von der gewünschten Form der Konfektionierung kann das erfindungsgemäße Mittel optional auch noch weitere Bestandteile oder Inhaltsstoffe enthalten.

Fettbestandteile im Mittel

Wenn das Mittel in Form einer Emulsion oder Creme zur Verfügung gestellt werden soll, hat sich der Einsatz mindestens eines Fettbestandteils als besonders vorteilhaft erwiesen. Bei den Fettbestandteilen handelt es sich um hydrophobe Substanzen, die in Gegenwart von Wasser unter Ausbildung von Mizell-Systemen Emulsionen formen können.

Unter "Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 12 C-Atomen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen.

Ganz besonders bevorzugt können die im Mittel zusätzlich eingesetzten Fettbestandteile ausgewählt werden aus der Gruppe der $C_{12}$-$C_{30}$-Fettalkohole, der $C_{12}$-$C_{30}$-Fettsäuretriglyceride, der $C_{12}$-$C_{30}$-Fett-säuremonoglyceride, der $C_{12}$-$C_{30}$-Fettsäurediglyceride und/oder der Kohlenwasserstoffe.

Im Rahmen einer weiteren bevorzugten Ausführungsform ist ein erfindungsgemäßes Mittel dadurch gekennzeichnet, dass es einen oder mehrere Fettbestandteile aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe enthält.

Als ganz besonders bevorzugte Fettbestandteile werden in diesem Zusammenhang die Bestandteile aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe verstanden. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Bei den $C_{12}$-$C_{30}$-Fettalkoholen kann es sich um gesättigte, ein- oder mehrfach ungesättigte, lineare oder verzweigte Fettalkohole mit 12 bis 30 C-Atomen handeln.

Beispiele für bevorzugte lineare, gesättigte $C_{12}$-$C_{30}$-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9Z)-Octadec-9-en-1-ol (Oleylalkohol), (9E)-Octadec-9-en-1-ol (Elaidylalkohol), (9Z,12Z)-Octadeca-9,12-dien-1-ol (Linoleylalkohol), (9Z,12Z,15Z)-Octadeca-9,12,15-trien-1-ol (Linolenoylalkohol), Gadoleylalkohol ((9Z)-Eicos-9-en-1-ol), Arachidonalkohol ((5Z,8Z,11Z,14Z)-Eicosa-5,8,11,14-tetraen-1-ol), Erucylalkohol ((13Z)-Docos-13-en-1-ol) und/oder Brassidylalkohol ((13E)-Docosen-1-ol).

Die bevorzugten Vertreter für verzweigte Fettalkohole sind 2-Octyl-dodecanol, 2-Hexyl-Dodecanol und/oder 2-Butyl-dodecanol.

Im Rahmen einer weiteren bevorzugten Ausführungsform ist ein erfindungsgemäßes Mittel dadurch gekennzeichnet, dass es einen oder mehrere $C_{12}$-$C_{30}$-Fettalkohole aus der Gruppe aus

Dodecan-1-ol (Dodecylalkohol, Laurylalkohol),
Tetradecan-1-ol (Tetradecylalkohol, Myristylalkohol),
Hexadecan-1-ol (Hexadecylalkohol, Cetylalkohol, Palmitylalkohol),
Octadecan-1-ol (Octadecylalkohol, Stearylalkohol),
Arachylalkohol (Eicosan-1-ol),
Heneicosylalkohol (Heneicosan-1-ol),
Behenylalkohol (Docosan-1-ol),
(9Z)-Octadec-9-en-1-ol (Oleylalkohol),

(9E)-Octadec-9-en-1-ol (Elaidylalkohol),
(9Z,12Z)-Octadeca-9,12-dien-1-ol (Linoleylalkohol),

(9Z,12Z,15Z)-Octadeca-9,12,15-trien-1-ol (Linolenoylalkohol),
Gadoleylalkohol ((9Z)-Eicos-9-en-1-ol),
Arachidonalkohol ((5Z,8Z,11Z,14Z)-Eicosa-5,8,11,14-tetraen-1-ol),
Erucylalkohol ((13Z)-Docos-13-en-1-ol),
Brassidylalkohol ((13E)-Docosen-1-ol),
2-Octyl-dodecanol,
2-Hexyl-Dodecanol und/oder
2-Butyl-dodecanol enthält.

Es hat sich als ganz besonders bevorzugt erwiesen, ein oder mehrere $C_{12}$-$C_{30}$-Fettalkohole in ganz bestimmten Mengenbereichen einzusetzen.

Des weiteren ist es ganz besonders bevorzugt, wenn die das Mittel - bezogen auf das Gesamtgewicht des Mittels - einen oder mehrere $C_{12}$-$C_{30}$-Fettalkohole in einer Gesamtmenge von 2,0 bis 50,0 Gew.-%, bevorzugt von 3,0 bis 30,0 Gew.-%, weiter bevorzugt von 4,0 bis 20,0 Gew.-%, noch weiter bevorzugt von 5,0 bis 15,0 Gew.-% und ganz besonders bevorzugt von 5,0 bis 10,0 Gew.-% enthält.

Weiterhin als ganz geeigneten Fettbestandteil kann das Mittel auch mindestens ein $C_{12}$-$C_{30}$-Fettsäuretriglycerid, der $C_{12}$-$C_{30}$-Fettsäuremonoglycerid und/oder $C_{12}$-$C_{30}$-Fettsäurediglycerid enthalten. Unter einem $C_{12}$-$C_{30}$-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte $C_{12}$-$C_{30}$-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure],

Arachidonsäure [(5Z,8Z,11Z,14Z)-Icosa-5,8,11,14-tetraensäure] und/oder Nervonsäure [(15Z)- Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem $C_{12}$-$C_{30}$-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die $C_{12}$-$C_{30}$-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)- Tetracos-15-ensäure].

Unter einem $C_{12}$-$C_{30}$-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Ganz besonders gute Ergebnisse wurden erhalten, wenn das Mittel mindestens ein $C_{12}$-$C_{30}$-Fettsäuremonoglycerid enthielt, welches ausgewählt ist aus den Monoestern von Glycerin mit einem Äquivalent Fettsäure aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Im Rahmen einer weiteren Ausführungsform ist ein erfindungsgemäßes Mittel dadurch gekennzeichnet, dass es mindestens ein $C_{12}$-$C_{30}$-Fettsäuremonoglycerid enthält, welches ausgewählt ist aus den Monoestern von Glycerin mit einem Äquivalent Fettsäure aus der Gruppe aus Dodecansäure, Tetradecansäure, Hexadecansäure, Tetracosansäure, Octadecansäure, Eicosansäure und/oder Docosansäure.

Es hat sich als bevorzugt erwiesen, ein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride (a4) in ganz bestimmten Mengenbereichen im Mittel einzusetzen.

Im Hinblick auf die Lösung der erfindungsgemäßen Aufgabenstellung hat es sich als vorteilhaft erwiesen, wenn das Mittel - bezogen auf das Gesamtgewicht des Mittels - ein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride (a4) in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthielt.

Im Rahmen einer ganz besonders bevorzugten Ausführungsform ist ein erfindungsgemäßes Verfahren dadurch gekennzeichnet, dass das Mittel - bezogen auf das Gesamtgewicht des Mittels - ein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthält.

Die $C_{12}$-$C_{30}$-Fettsäuremono-, $C_{12}$-$C_{30}$-Fettsäuredi- und/oder $C_{12}$-$C_{30}$-Fettsäuretriglyceride können als alleinige Fettbestandteile (a4) in den Mitteln eingesetzt werden. Es kann sich jedoch auch erfindungsgemäß, mindestens ein $C_{12}$-$C_{30}$-Fettsäuremono-, $C_{12}$-$C_{30}$-Fettsäuredi- und/oder $C_{12}$-$C_{30}$-Fettsäuretriglycerid in Kombination mit mindestens einem $C_{12}$-$C_{30}$-Fettalkohol in das Mittel einzuarbeiten.

Weiterhin als ganz besonders bevorzugten Fettbestandteil kann das Mittel auch mindestens einen Kohlenwasserstoff enthalten.

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Als besonders geeignet haben sich in diesem Zusammenhang flüssige Paraffinöle (Paraffinum Liquidum und Paraffinium Perliquidum) erwiesen. Ganz besonders bevorzugt handelt es sich bei dem Kohlenwasserstoff um Paraffinum Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Ganz besonders gute Ergebnisse wurden erhalten, wenn das Mittel mindestens einen Kohlenwasserstoff enthielt, der ausgewählt ist aus der Gruppe der Mineralöle, der flüssige Paraffinöle, Isoparaffinöle, halbfeste Paraffinöle, Paraffinwachse, Hartparaffin (Paraffinum Solidum), Vaseline und Polydecene.

Im Rahmen einer ganz besonders bevorzugten Ausführungsform ist ein erfindungsgemäßes Mittel dadurch gekennzeichnet, dass es mindestens einen Fettbestandteil aus der Gruppe der Kohlenwasserstoffe enthält.

Im Hinblick auf die Lösung der erfindungsgemäßen Aufgabenstellung hat es sich als ganz besonders bevozugt erwiesen, wenn das Mittel - bezogen auf das Gesamtgewicht des Mittels - einen oder mehrere Kohlenwasserstoffe in einer Gesamtmenge von 0,5 bis 20,0 Gew.-%, bevorzugt 0,7 bis 10,0 Gew.-%, weiter bevorzugt von 0,9 bis 5,0 Gew.-% und ganz besonders bevorzugt von 1,0 bis 4,0 Gew.-% enthielt.

<u>Wassergehalt im Mittel</u>

Bei dem zuvor beschriebenen Mittel handelt es sich um ein anwendungsbereites Mittel, welches auf das keratinische Material appliziert werden kann. Dieses anwendungsbereite Mittel besitzt bevorzugt einen hohen Wasseranteil. Es hat sich herausgestellt, dass besonders die Mittel besonders gut geeignet sind, die - bezogen auf das Gesamtgewicht des Mittels - 50,0 bis 98,0 Gew.-%, bevorzugt 60,0 bis 90,0 Gew.-%, weiter bevorzugt 70,0 bis 90,0 Gew.-% und ganz besonders bevorzugt 75,0 bis 90,0 Gew.-% Wasser enthalten.

In einer weiteren explizit ganz besonders bevorzugten Ausführungsform ist ein erfindungsgemäßes Mittel dadurch gekennzeichnet, dass es - bezogen auf das Gesamtgewicht des Mittels - 50,0 bis 98,0 Gew.-%, bevorzugt 60,0 bis 90,0 Gew.-%, weiter bevorzugt 70,0 bis 90,0 Gew.-% und ganz besonders bevorzugt 75,0 bis 90,0 Gew.-% Wasser enthält.

<u>weitere Tenside im Mittel</u>

Bedingt durch den optionalen, aber bevorzugten Gehalt an Wasser und Fettbestandteil liegt das erfindungsgemäße Mittel besonders bevorzugt in Form einer Emulsion vor. Um die Ausbildung der Emulsion weiter zu optimieren, kann es sich als bevorzugt erweisen, im Mittel weiterhin mindestens ein weiteres Tensid einzusetzen, einzusetzen.

Unter dem Begriff Tenside (T) werden grenzflächenaktive Substanzen, die an Ober- und Grenzflächen Adsorptionsschichten bilden oder in Volumenphasen zu Mizell-Kolloiden oder lyotropen Mesophasen aggregieren können, verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Im Rahmen einer ganz besonders bevorzugten Ausführungsform ist ein erfindungsgemäßes Mittel dadurch gekennzeichnet, dass es mindestens ein nichtionisches Tensid enthält.

Nichtionische Tenside enthalten beipsielsweise als hydrophile Gruppe eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,

- mit einem Methyl- oder $C_2$ - $C_6$ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol® - Typen (Cognis),
- alkoxilierte Triglyceride,
- alkoxilierte Fettsäurealkylester der Formel (Tnio-1)

$$R^1CO\text{-}(OCH_2CHR^2)_wOR^3 \qquad \text{(Tnio-1)}$$

  in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Anlagerungsprodukte von Ethylenoxid an Fettsäurealkanolamide und Fettamine,

Weitere typische Beispiele für nichtionische Tenside sind Fettsäureamidpolyglycolether, Fettaminpolyglycolether, Mischether bzw. Mischformale, Proteinhydrolysate (insbesondere pflanzliche Produkte auf Weizenbasis) und Polysorbate.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure sowie die Zuckertenside erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Besonders gute Ergebnisse im Hinblick auf die weitere Verbesserung der Farbintensität wurden erhalten, wenn das erfindungsgemäße Mittel zusätzlich zu den nichtionischen Tensiden (a1) ein weiteres nichtionisches Tensid (a4) enthielt. Bei diesem zusätzlichen nichtionischen Tensid (a4) handelt es sich ganz besonders bevorzugt um ein Tensid der Formel (E-II),

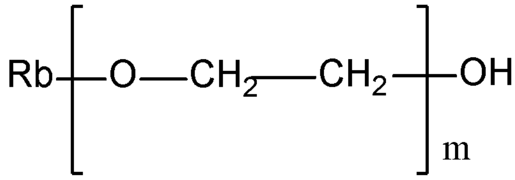

(E-II)

worin

Rb für eine gesättigte oder ungesättigte, unverzweigte oder verzweigte $C_{12}$-$C_{24}$-Alkylgruppe steht und und
m für eine ganze Zahl von 20 bis 40, bevorzugt für eine ganze Zahl von 20 bis 35 und besonders bevorzugt für die Zahl 30, steht.

In einer weiteren ganz besonders bevorzugten Ausführungsform ist ein erfindungsgemäßes Mittel dadurch gekennzeichnet, dass es zusätzlich mindestens einen weiteren nichtionischen Emulgator (a4) der Formel (E-II) enthält,

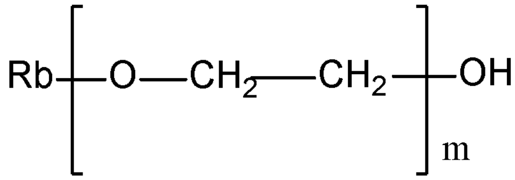

(E-II)

worin

Rb für eine gesättigte oder ungesättigte, unverzweigte oder verzweigte $C_{12}$-$C_{24}$-Alkylgruppe steht und und
m für eine ganze Zahl von 20 bis 40, bevorzugt für eine ganze Zahl von 20 bis 35 und besonders bevorzugt für die Zahl 30, steht.

Bei einem besonders gut geeigneten nichtionischen Tensid dieses Typs handelt es sich um Ceteareth-30. Bei Ceteareth-30 handelt es sich um ein Gemisch aus Cetylalkohol und Stearylalkohol, die jeweils mit 30 Einheiten Ethylenoxid ethoxyliert sind. Das Gemisch aus Cetylalkohol und Stearylalkohol wird als Cetearylalkohol bezeichnet. Ceteareth-30 besitzt die CAS-Nummer 68439-49-6 und ist beispielsweise unter dem Handelsnamen Eumulgin B3 von der Firma BASF käuflich zu erwerben.

Weiterhin besonders gute Ergebnisse im Hinblick auf die Verbesserung der Farbintensität wurden erhalten, wenn das erfindungsgemäße Mittel zusätzlich zu den nichtionischen Tensiden (a1) ein weiteres nichtionisches Tensid (a5) enthielt. Bei diesem zusätzlichen nichtionischen Tensid (a5) handelt es sich ganz besonders bevorzugt um ein Tensid der Formel (E-III),

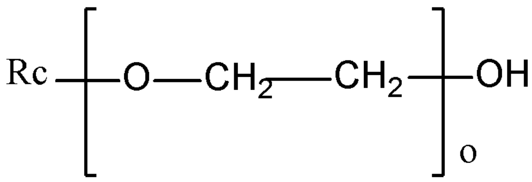

(E-III)

worin

Rc für eine gesättigte oder ungesättigte, unverzweigte oder verzweigte $C_{12}$-$C_{24}$-Alkylgruppe steht und und
o für eine ganze Zahl von 80 bis 120, bevorzugt für eine ganze Zahl von 90 bis 110 und besonders bevorzugt für die Zahl 100, steht.

In einer weiteren ganz besonders bevorzugten Ausführungsform ist ein erfindungsgemäßes Mittel dadurch gekennzeichnet, dass es dass es zusätzlich mindestens einen weiteren nichtionischen Emulgator (a5) der Formel (E-III) enthält,

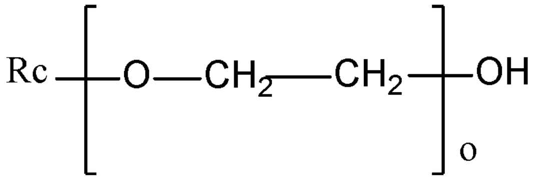

(E-III)

worin

Rc für eine gesättigte oder ungesättigte, unverzweigte oder verzweigte $C_{12}$-$C_{24}$-Alkylgruppe steht und und
o für eine ganze Zahl von 80 bis 120, bevorzugt für eine ganze Zahl von 90 bis 110 und besonders bevorzugt für die Zahl 100, steht.

Ein besonders gut geeignetes nichtionisches Tensid dieses Typs trägt den Handelsnamen Brij S 100 bzw. Brij S 100 PA SG. Hierbei handelt es sich um Stearylalkohol, ethoxyliert mit 100 EO, der von der Firma Croda kommerziell erhältlich ist und die CAS-Nummer 9005-00-9 trägt.

Es hat sich als ganz besonders bevorzugt erwiesen, wenn das Mittel zusätzlich sowohl mindestens ein nichtionisches Tensid der Formel (E-I) als auch mindestens ein nichtionisches Tensid der Formel (E-II) enthält.

Lösungsmittel im Mittel

Der Einsatz von Lösungsmitteln hat weiterhin zu sehr guten Ergebnissen geführt. Aus diesem Grund kann das erfindungsgemäße Mittel als optionalen Bestandteil zusätzlich mindestens ein Lösungmittel enthalten.

Als geeignete Lösungsmittel können beispielsweise Lösungsmittel aus der Gruppe aus 1,2-Propylenglycol, 1,3-Propylenglycol, Ethylenglycol, 1,2- Butylenglycol, Dipropylenglycol, Ethanol, Isopropanol, Diethylenglycolmonoethylether, Glycerin, Phenoxyethanol und Benzylalkohol eingesetzt werden. Der Einsatz von 1,2-Propylenglycol ist ganz besonders bevorzugt.

In einer weiteren ganz besonders bevorzugten Ausführungsform ist ein erfindungsgemäßes Mittel dadurch gekennzeichnet, dass es mindestens ein Lösungsmittel aus der Gruppe aus 1,2-Propylenglycol, 1,3-Propylenglycol, Ethylenglycol, 1,2- Butylenglycol, Dipropylenglycol, Ethanol, Isopropanol, Diethylenglycolmonoethylether, Glycerin, Phenoxyethanol und Benzylalkohol, ganz besonders bevorzugt 1,2-Propylenglycol, enthält.

1,2-Propylenglycol wird alternativ auch als 1,2-Propandiol bezeichnet wird und trägt die CASNummern 57-55-6 [(RS)-1,2-Dihydroxypropan], 4254-14-2 [(R)-1,2-Dihydroxypropan] und 4254-153 [(S)-1,2-Dihydroxypropan]. Ethylenglycol wird alternativ auch als 1,2-Ethandiol bezeichnet und trägt die CAS-Nummer 107-21-1.Glycerin wird alternativ auch als 1,2,3-Propantriol bezeichnet und trägt die CAS-Nummer 56-81-5. Phenoxyethanol besitzt die Cas-Nummer 122-99-6.

Alle zuvor beschriebenen Lösungsmittel sind bei verschiedenen Chemikalien-Lieferanten wie beispielsweise Aldrich oder Fluka kommerziell erhältlich.

Durch Verwendung der vorgenannten Lösungsmittel in geeigneten Einsatzmengen kann ein besonders stabiles Mittel erhalten werden, mit dem sich auf dem keratinischen Material Farbergebnisse mit ganz besonders hoher Intensität erhalten lassen.

In einer weiteren bevorzugten Ausführungsform ist ein erfindungsgemäßes Mittel dadurch gekennzeichnet, dass es - bezogen auf das Gesamtgewicht des Mittels- ein oder mehrere Lösungsmittel in einer Gesamtmenge von 1,0 bis 20,0 Gew.-%, bevorzugt 2,0 bis 15,0 Gew.-%, weiter bevorzugt 3,0 bis 15,0 Gew.-% und ganz besonders bevorzugt 4,0 bis 10,0 Gew.% 1,2-Propylenglycol enthält.

Polymere im Mittel

Das erfindungsgemäße Mittel kann zusätzlich auch mindestens ein filmbildendes Polymer enthalten. Das filmbildende Polymer kann zum Beispiel ausgewählt ist aus der Gruppe aus Polyvinylpyrrolidon (PVP), Vinylpyrrolidon/-Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, explizit ganz besonders bevorzugt Polyvinylpyrrolidon (PVP).

Weitere geeignete filmbildende Polymere können augewählt sein aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der

Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Es haben sich insbesondere die filmbildenden Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer $C_1$-$C_{20}$-Alkylgruppe, einer Arylgruppe oder einer C2-C10-Hydroxyalkylgruppe.

Weitere filmbildende Polymere können ausgewählt sein aus den Homo- oder Copolymeren von Isooctyl-(meth) acrylat; Isononyl-(meth)acrylat; 2-Ethylhexyl-(meth)acrylat; Lauryl-(meth)acrylat); isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Weitere geeignete anionische Copolymere sind beispielsweise Copolymere aus Acrylsäure, Methacrylsäure oder deren $C_1$-$C_6$-Alkylestern, wie sie unter der INCI-Deklaration Acrylates Copolymere vertrieben werden. Ein geeignetes Handelsprodukt ist beispielsweise Aculyn® 33 der Firma Rohm & Haas. Weiterhin bevorzugt sind aber auch Copolymere aus Acrylsäure, Methacrylsäure oder deren $C_1$-$C_6$-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche Polymere sind beispielsweise Aculyne 22 (Acrylates/Steareth-20 Methacrylate Copolymer), Aculyne 28 (Acrylates/Beheneth-25 Methacrylate Copolymer), Structure 2001®(Acryla-tes/Steareth-20 Itaconate Copolymer), Structure 3001®(Acrylates/Ceteth-20 Itaconate Copolymer), Structure Plus®(Acrylates/Aminoacrylates C10-30 Alkyl PEG-20 Itaconate Copolymer), Carbopol® 1342, 1382, Ultrez 20, Ultrez 21 (Acrylates/C10-30 Alkyl Acrylate Crosspolymer), Synthalen W 2000® (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin geeignet sind die Copolymere Octylacrylamid/acrylates/ butylaminoethyl-methacrylate copolymer, wie es beispielsweise unter den Handelsnamen AMPHOMER® ou LOVOCRYL® 47 von NATIONAL STARCH kommerziell vertrieben wird, oder auch die Copolymere von Acrylates/Octylacrylamide die unter den Handelsnamen DERMACRYL® LT und DERMACRYL® 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildende hydrophobe Polymere die Block-Copoylmere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/-Butadien. Entsprechende Polymere werden von der BASF unter dem Handelsnamen "Luvitol HSB" kommerziell vertrieben.

Die Mittel können ferner auch noch weitere Wirk-, Hilfs- und Zusatzstoffe enthalten, wie beispielsweise Lösungsmittel, Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, $N_2O$, Dimethylether, $CO_2$ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

pH-Wert der Mittel

Die pH-Werte des erfindungsgemäßen Mittels wird bevorzugt auf einen leicht sauren bis alkalischen pH-Wert eingestellt. Ganz besonders bevorzugt besitzt das Mittel einen alkalischen pH-Wert im Bereich von 4,0 bis 11,5 bevorzugt von 5,0 bis 11,0.

Zur Einstellung des gewünschten pH-Wertes kann das Mittel (a) und/oder (b) mindestens ein Alkalisierungsmittel enthalten. Bei den pH-Werten im Sinne der vorliegenden Erfindung handelt es sich um pH-Werte, die bei einer Temperatur von 22°C gemessen wurden.

Als Alkalisierungsmittel können die Mittel beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -$SO_3H$-Gruppe enthält. Bevorzugte Aminosäuren sind Aminocarbonsäuren, insbesondere $\alpha$-(alpha)-Aminocarbonsäuren und $\omega$-Aminocarbonsäuren, wobei $\alpha$-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische $\alpha$-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

In einer weiteren ganz besonders bevorzugten Ausführungsform ist ein erfindungsgemäßes Verfahren dadurch gekennzeichnet, dass das Färbemittel (a) mindestens ein Alkalisierungsmittel aus der Gruppe aus Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat enthält.

Zur Einstellung des gewünschten pH-Wertes kann das erfindungsgemäße Mittel auch mindestens ein ein Puffersystem aus mindestens einer anorganischen oder organischen Säure und mindestens einem Salz dieser Säure enthalten.

Eine ganz besonders gut geeignete anorganische Säure ist hierbe Kaliumdihydrogenphosphat Kaliumdihydrogenphosphat besitzt die Summenformel $KH_2PO_4$ und trägt die CAS-Nummer 7778-77-0. Kaliumdihydrogenphosphat besitzt eine molare Masse von 136,09 g/mol. Es ist gut löslich in Wasser (222 g/l bei 20 °C) und reagiert in Wasser sauer. Eine 5 %ige Lösung von Kaliumdihydrogenphosphat in Wasser besitzt einen pH-Wert von 4,4.

Eine weitere ganz besonders gut geeignete anorganische Säure (b2-I) ist Natriumdihydrogenphosphat. Natriumdihydrogenphosphat besitzt die Summenformel $NaH_2PO_4$ und trägt die CAS-Nummern 7558-80-7 (Anhydrat), 10049-21-5 (Monohdrat) und 13472-35-0 (Dihydrat). Das wasserfreie Natriumdihydrogenphosphat besitzt eine molare Masse von 119,98 g/mol. Natriumdihydrogenphosphat reagiert in wässriger Lösung sauer.

Besonders bevorzugt als korrespondierendes Salz der vorgenannten beiden Säuren ist Dikaliumhydrogenphosphat. Dikaliumhydrogenphosphat besitzt die Summenformel $K_2HPO_4$ und trägt die CAS-Nummern 7758-11-4 (wasserfrei) und 16788-57-1 (Trihydrat). Das wasserfreie Dikaliumhydrogenphosphat besitzt eine molare Masse von 174,18 g/mol. Dikaliumhydrogenphosphat reagiert in wässriger Lösung alkalisch.

Ebenfalls besonders bevorzugt als korresprondierdens Salz der vorgenannten beiden Säuren (b2-II) ist Dinatriumhydrogenphosphat. Dinatriumhydrogenphosphat besitzt die Summenformel $Na_2HPO_4$ und trägt die CAS-Nummern 7558-79-4 (wasserfrei), 10028-24-7 (Dihydrat), 7782-85-6 (Heptahydrat) und 10039-32-4 (Dodecahydrat). Das wasserfreie Dinatriumhydrogenphosphat besitzt eine molare Masse von 141,96 g/mol. Dinatriumhydrogenphosphat reagiert in wässriger Lösung alkalisch.

Verfahren zum Färben von Keratinmaterial

Die zuvor beschriebenen Mittel lassen sich hervorragend in Verfahren zum Färben von keratinischem Material, insbesondere von menschlichen Haaren, einsetzen.

Ein zweiter Gegenstand der vorliegenden Erfindung ist daher ein Verfahren zum Färben von keratinischem Material, insbesondere menschlichen Haaren, bei welchem ein Mittel, wie es bei der Beschreibung des ersten Erfindungsgegenstands im Detail offenbart wurde, auf die keratinischen Fasern aufgetragen wird und gegebenenfalls nach einer Einwirkzeit von 30 Sekunden bis 45 Minuten wieder ausgespült wird.

Mit anderen Worten ist ein zweiter Gegenstand der Erfindung ein Verfahren zum Färben von keratinischen Fasern, insbesondere menschlichen Haaren, umfassend die folgenden Schritte:

(1) Anwendung eines Färbemittels auf dem keratinischem Material, wobei das Färbemittel ein Mittel ist, wie es bei der Beschreibung der ersten Erfindungsgegenstand im Detail offenbart wurde,
(2) Einwirken des Färbemittels auf dem keratinischen Material und
(3) Ausspülen des Färbemittels mit Wasser.

In Schritt (1) des erfindungsgemäßen Verfahrens wird das Mittel des ersten Erfindungsgegenstand auf dem keratinische Material, bei dem es sich ganz besonders bevorzugt um menschliche Haare handelt, angewendet.

In Schritt (2) des erfindungsgemäßen Verfahrens wird das Mittel dann nach seiner Applikation auf das keratinische Material einwirken gelassen. In diesem Zusammenhang sind verschiedene Einwirkzeiten von beispielsweise 30 Sekunden bis 60 Minuten denkbar.

Ein großer Vorteil des erfindungsgemäßen Färbesystems liegt jedoch darin, dass auch in sehr kurzen Zeiträumen nach kurzen Einwirkzeiten ein intensive Farbergebnis erzielt werden kann. Aus diesem Grund ist es von Vorteil, wenn die Anwendungsmischung nach ihrer Applikation nur für vergleichsweise kurze Zeiträume von 30 Sekunden bis 15 Minuten, bevorzugt von 30 Sekunden bis 10 Minuten, und besonders bevorzugt von 1 bis 5 Minuten auf dem Keratinmaterial verbleibt.

In einer weiteren bevorzugten Ausführungsform ist ein erfindungsgemäßes Verfahren gekennzeichnet durch das
(2) Einwirken des Färbemittels auf dem keratinischen Material für einen Zeitraum von 30 Sekunden bis 15 Minuten, bevorzugt von 30 Sekunden bis 10 Minuten, und besonders bevorzugt von 1 bis 5 Minuten.

Im Anschluss an das Einwirken der Anwendungsmischung auf das Keratinmaterial wird diese schließlich in Schritt (3) des Verfahrens mit Wasser ausgespült.

Hierbei kann die Awendungsmischung in einer Ausführungsform nur mit Wasser, d.h. ohne Zuhilfenahme eines

Nachbehandlungsmittels oder eines Shampoos, ausgewaschen werden. Auch die Anwendung eines Nachbehandlungsmittels oder Conditioners in Schritt (6) ist prinzipiell denkbar.

Zur Lösung der erfindungsgemäßen Aufgabenstellung und zur Erhöhung des Anwendungskomforts hat es sich jedoch als ganz besonders bevorzugt herausgestellt, das Ausspülen des Mittels in Schritt (3) ausschließlich mit Wasser ohne Zuhilfenahme eines weiteren Nachbehandlungsmittels, Shampoos oder Conditioners vorzunehmen.

In einer weiteren bevorzugten Ausführungsform ist ein erfindungsgemäßes Verfahren gekennzeichnet durch das

(3) Ausspülen des Färbemittels ausschließlich mit Wasser.

Verfahren zum Färben von Keratinmaterial, bei welchem zunächst das anwendungsbereite Mittel hergestellt wird.

Wie bereits zuvor beschrieben, handelt es sich bei dem Mittel des ersten Erfindungsgegenstands um ein anwendungsbereits Mittel, das dem Anwender entweder direkt in seiner anwendungsbereiten Form zur Verfügung gestellt wird, oder aber das erst kurz vor der Anwendung durch Vermischen von verschiedenen Mitteln hergestellt wird.

Um eine besonders feine Verteilung der Pigmente zu gewährleisten, hat es sich als ganz besonders bevorzugt herausgestellt, das anwendungsbereite Mittel kurz vor der Anwendung durch Vermischen von zwei oder drei verschiedenen Mitteln herzustellen.

Im Rahmen einer besonders bevorzugten Ausführungsform wird das anwendungsbereite Mittel demnach durch Vermischen von mindestens zwei verschiedenen Mitteln hergestellt, wobei das erste dieser beiden Mittel das Gemisch aus nichtionischem Emulgator (a1) und Pigment(en) (a2) umfasst. So kann das Gemisch aus nichtionischem Emulgator (a1) und Pigment(en) (a2) beispielsweise eine Vordispersion darstellen, die in Form eines Konzentrats zur Verfügung gestellt wird. Das zweite Mittel enthält mindestens ein aminofunkionalisiertes Silikonpolymer (a3) und kann beispielsweise eine wasserhaltige, kosmetische Trägerformulierung darsellen. Es ist ebenfalls erfindungsgemäß, wenn das erste Mittel, welches das Gemisch aus nichtionischem Emulgator (a1) und Pigment(en) (a2) umfasst, eine wasserhaltige kosmetische Trägerformulierung darstellt und das zweite Mittel, welches das aminofunkionalisiertes Silikonpolymer (a3) beinhaltet, in Form eines Konzentrats vorliegt. Zur Herstellung des anwendungsbereiten Mittes werden die beiden vorgenannten Mittel dann miteinander verschüttelt oder verrührt.

Ein weiterer Gegenstand der vorliegenden Anmeldung ist daher ein Verfahren zum Färben von keratinischem Material, insbesondere menschlichen Haaren, umfassend die folgenden Schritte:

(1) Bereitstellung eines Mittels (I), wobei das Mittel (I) enthält:

(a1) mindestens einen nichtionischen Emulgator mit einem HLB-Wert von 4,0 bis 12,0, und
(a2) mindestens ein Pigment,

(2) Bereitstellung eines Mittels (II), wobei das Mittel (II) enthält:
(a3) mindestens ein aminofunktionalisiertes Silikonpolymer, und
(3) Herstellung einer Anwendungsmischung durch Vermischen des Mittels (I) mit dem Mittel (II),
(4) Applizieren der in Schritt (3) hergestellten Anwendungsmischung auf dem keratinischen Material,
(5) Einwirken der in Schritt (4) applizierten Anwendungsmischung auf dem keratinischen Material und
(6) Ausspülen der Anwendungsmischung mit Wasser,

wobei die Inhaltsstoffe (a1), (a2) und (a3) bei der Beschreibung des ersten Erfindungsgegenstands bereits im Detail offenbart wurden.

Die optional zusätzlich anwendbaren nichtionischen Emulgatoren (a4) und/oder (a5) können im Rahmen dieser Ausführungsform beispielsweise im Mittel (I) und/oder im Mittel (II) enthalten sein.

Bevorzugt ist daher ein Verfahren zum Färben von keratinischem Material, insbesondere menschlichen Haaren, umfassend die folgenden Schritte:

(1) Bereitstellung eines Mittels (I), wobei das Mittel (I) enthält:

(a1) mindestens einen nichtionischen Emulgator mit einem HLB-Wert von 4,0 bis 12,0, und
(a2) mindestens ein Pigment,
(a4) mindestens einen nichtionischen Emulgator der Formel (E-II), und
(a5) mindestnes einen nichtionischen Emulgator der Formel (E-III)

(2) Bereitstellung eines Mittels (II), wobei das Mittel (II) enthält:
(a3) mindestens ein aminofunktionalisiertes Silikonpolymer, und

(3) Herstellung einer Anwendungsmischung durch Vermischen des Mittels (I) mit dem Mittel (II),
(4) Applizieren der in Schritt (3) hergestellten Anwendungsmischung auf dem keratinischen Material,
(5) Einwirken der in Schritt (4) applizierten Anwendungsmischung auf dem keratinischen Material und
(6) Ausspülen der Anwendungsmischung mit Wasser,

wobei die Inhaltsstoffe (a1), (a2), (a3), (a4) und (a5) bei der Beschreibung des ersten Erfindungsgegenstands bereits im Detail offenbart wurden.

Ganz besonders bevorzugt ist ein Verfahren zum Färben von keratinischem Material, insbesondere menschlichen Haaren, umfassend die folgenden Schritte:

(1) Bereitstellung eines Mittels (I), wobei das Mittel (I) enthält:

(a1) mindestens einen nichtionischen Emulgator der Formel (E-I), und
(a2) mindestens ein Pigment, und
(a4) mindestens einen nichtionischen Emulgator der Formel (E-II), und
(a5) mindestens einen nichtionischen Emulgator der Formel (E-III)

(2) Bereitstellung eines Mittels (II), wobei das Mittel (II) enthält:
(a3) mindestens ein aminofunktionalisiertes Silikonpolymer, und
(3) Herstellung einer Anwendungsmischung durch Vermischen des Mittels (I) mit dem Mittel (II),
(4) Applizieren der in Schritt (3) hergestellten Anwendungsmischung auf dem keratinischen Material,
(5) Einwirken der in Schritt (4) applizierten Anwendungsmischung auf dem keratinischen Material und
(6) Ausspülen der Anwendungsmischung mit Wasser,

wobei die Inhaltsstoffe (a1), (a2), (a3), (a4) und (a5) bei der Beschreibung des ersten Erfindungsgegenstands bereits im Detail offenbart wurden.

Abhängig vom gewählten pH-Wert hat das Aminosilikon (a3) in eingen Fällen eine verringerte Lagerstabilität in wässrigem Milieu gezeigt. In diesen Fällen kann es von Vorteil sein, auch das aminofunktionalisiertes Silikonpolymer (a3) in einem separaten Mittel zu konfektionieren und sowohl die Inhaltssoffe (a1) und (a2) als auch das Aminosilikon (a3) erst kurz vor der Anwendung mit einer Basis-Formulierung zu vermischen. In diesem Fall werden zur Herstellung des anwendungsbereiten Färbemittels mindestens drei verschiedene Mittel miteinander vermischt.

Besonders bevorzugt ist daher ein Verfahren zum Färben von keratinischem Material, insbesondere menschlichen Haaren, umfassend die folgenden Schritte:

(1) Bereitstellung eines Mittels (I), wobei das Mittel (I) enthält:

(a1) mindestens einen nichtionischen Emulgator mit einem HLB-Wert von 4,0 bis 12,0, und
(a2) mindestens ein Pigment,

(2) Bereitstellung eines Mittels (II), wobei das Mittel (II) enthält:
(a3) mindestens ein aminofunktionalisiertes Silikonpolymer, und
(3) Bereitstellung eines Mittels (III), wobei das Mittel (III) enhält:

(a4) mindestens einen nichtionischen Emulgator (a4) der Formel (E-II) und/oder
(a5) mindestens einen nichtionischen Emulgator (a5) der Formel (E-III),

(4) Herstellung einer Anwendungsmischung durch Vermischen des Mittels (I) mit dem Mittel (II) und gegebenenfalls mit dem Mittel (III),
(5) Applizieren der in Schritt (4) hergestellten Anwendungsmischung auf dem keratinischen Material,
(6) Einwirken der in Schritt (5) applizierten Anwendungsmischung auf dem keratinischen Material und
(7) Ausspülen der Anwendungsmischung mit Wasser,

wobei die Inhaltsstoffe (a1), (a2), (a3), (a4) und (a5) bei der Beschreibung des ersten Erfindungsgegenstands bereits im Detail offenbart wurden.

Das Mittel (III) stellt hierbei eine kosmetische Träger-Formulierung oder eine Basis-Creme dar, welche die Emulgatoren (a4) und/oder (a5) enthält.

Zusammenfassend ist ein weiterer Gegenstand der vorliegenden Anmeldung ein Verfahren zum Färben von keratinischem Material, insbesondere menschlichen Haaren, umfassend die folgenden Schritte:

(1) Bereitstellung eines Mittels (I), wobei das Mittel (I) enthält:

(a1) mindestens einen nichtionischen Emulgator mit einem HLB-Wert von 4,0 bis 12,0, und
(a2) mindestens ein Pigment,

(2) Bereitstellung eines Mittels (II), wobei das Mittel (II) enthält:
(a3) mindestens ein aminofunktionalisiertes Silikonpolymer, und
(3) gegebenenfalls Bereitstellung eines Mittels (III), wobei das Mittel (III) enhält:

(a4) mindestens einen nichtionischen Emulgator (a4) der Formel (E-II) und/oder
(a5) mindestens einen nichtionischen Emulgator (a5) der Formel (E-III),

(4) Herstellung einer Anwendungsmischung durch Vermischen des Mittels (I) mit dem Mittel (II) und gegebenenfalls mit dem Mittel (III),
(5) Applizieren der in Schritt (4) hergestellten Anwendungsmischung auf dem keratinischen Material,
(6) Einwirken der in Schritt (5) applizierten Anwendungsmischung auf dem keratinischen Material und
(7) Ausspülen der Anwendungsmischung mit Wasser,

wobei die Inhaltsstoffe (a1), (a2), (a3), (a4) und (a5) bei der Beschreibung des ersten Erfindungsgegenstands bereits im Detail offenbart wurden.

Im Rahmen dieser Ausführungsform stellt das Mittel (I) bevorzugt eine Vordispersion der Pigmente (a2) in dem oder den nichtionischen Tensiden (a1) dar, die beispielsweise in Form eines Konzentrats vorliegen kann. Das Mittel (II) ist bevorzugt ebenfalls ein Konzentrat, welches das oder die aminofunktionalisierten Silikonpolymere (a3) enthält.

Vor der Anwendung werden dann die beiden Mittel bzw. Konzentrate (I) und (II) mit der Trägerformulierung (III) vermischt. Die kosmetische Träger-Formulierung bzw. das Mittel (III), enthält im Rahmen dieser Ausführungsform die nichtionischen Emulgatoren (a4) und/oder (a5).

Die Reihenfolge des Vermischens ist hierbei beliebig. So können zunächst die Mittel (I) und (II) minteinander vermischt werden, woraufhin diese Mischung dann mit dem Mittel (III) vermischt wird. Ebenso ist es denkbar, zunächst die Mittel (II) und (III) zu vermischen und diese Mischung dann mit dem Mittel (I) zu vermischen. Auch können alle drei Mittel (I), (II) und (III) zusammengegeben und denn erst durch Schütteln oder Rühren vermischt werden.

Die Trägerformulierung ist wasserhaltig und besitzt bevorzugt einen hohen Wassergehalt. Die optional einsatzbaren weiteren Inhaltsstoffe des ersten Erfindungsgegenstands können auch in dieser Trägerformulierung enthalten sein.

Schließlich kann es sich im Rahmen einer weiteren Ausführungsform als vorteilhaft erweisen, auch die Bestandteile (a1) und (a2) voneinander zu trennen, so dass mit der Herstellung des anwendungsbereiten Mittels die drei zuvor voneinander getrennten Bestandteile (a1), (a2) und (a3) miteinaner vermischt werden. Diese Herstellungform kann beispielsweise dann von Vorteil sein, wenn das oder die Pigmente (a2) in geringeren Mengen und/oder in Form eines Pulvers eingesetzt werden sollen. Die Konfektionierung der Pigmente in Pulverform vereinfacht die quantitative Überführung der Pigmente in ein Mischgefäß oder einen anderen Container.

Ein weiterer Gegenstand der vorliegenden Anmeldung ist damit ein Verfahren zum Färben von keratinischem Material, insbesondere menschlichen Haaren, umfassend die folgenden Schritte:

(1) Bereitstellung eines Mittels (I), wobei das Mittel (I) enthält:
(a1) mindestens einen nichtionischen Emulgator mit einem HLB-Wert von 4,0 bis 12,0,
(2) Bereitstellung eines Mittels (II), wobei das Mittel (II) enthält:
(a2) mindestens Pigment,
(3) Bereitstellung eines Mittels (III), wobei das Mittel (III) enthält:
(a3) mindestens ein aminofunktionalisiertes Silikonpolymer,
(4) Herstellung einer Anwendungsmischung durch Vermischen der Mittel (I) und (II) und (III),
(5) Applizieren der in Schritt (4) hergestellten Anwendungsmischung auf dem keratinischen Material,
(6) Einwirken der in Schritt (5) applizierten Anwendungsmischung auf dem keratinischen Material und
(7) Ausspülen der Anwendungsmischung mit Wasser,

wobei die Inhaltsstoffe (a1), (a2) und (a3) bei der Beschreibung des ersten Erfindungsgegenstands bereits im Detail offenbart wurden.

Hierbei enthält das Mittel (I) mindestens nichtionischen Emulgator (a1) und kann beispielsweise in Form eines Konzentrats vorliegen. Es ist ebenfalls erfindungsgemäß, wenn das Mittel (I) in Form einer Träger-Formulierung oder einer Träger-Creme vorliegt, welche - neben weiteren optionalen Bestandteilen, den nichtionischen Emulgator (a1)

enthält. Im Rahmen dieser Ausführungsform können auch die nichtionischen Emulgatoren (a4) und/oder (a5) in diesem Mittel enthalten sein.

Das Mittel (II) enthält mindestens ein Pigment (a2). In einer möglichen Form der Konfektionierung wird das Pigment beipsielsweise in Form eines Pulvers bereitgestellt.

Das Mittel (III) enthält mindestens ein aminofunktionalisiertes Silikonpolymer (a3) und liegt bevorzugt in Form eines Konzentrats vor.

Mehrkomponenten-Verpackungseinheit

Zur Erhöhung des Anwenderkomforts können dem Anwender die zuvor beschriebenen Mittel in Form einer Mehrkomponenten-Verpackungseinheit zur Verfügung gestellt werden.

Ein weiterer Gegenstand ist daher eine Mehrkomponenten-Verpackungseinheit (Kit-of-parts) zum Färben von keratinischem Material, umfassend getrennt voneinander konfektioniert

- einen ersten Container mit einem Mittel (I), wobei das Mittel (I) enthält:

  (a1) mindestens einen nichtionischen Emulgator mit einem HLB-Wert von 4,0 bis 12,0,
  (a2) mindestens ein Pigment, und

- einen zweiten Container mit einem Mittel (II), wobei das Mittel (II) enthält:
  (a3) mindestens ein aminofunktionalisiertes Silikonpolymer,

wobei die Inhaltsstoffe (a1), (a2) und (a3) bei der Beschreibung des ersten Erfindungsgegenstands bereits im Detail offenbart wurden.

Im Rahmen dieser Ausführungsform bevorzugt ist eine Mehrkomponenten-Verpackungseinheit (Kit-of-parts), umfassend getrennt konfektioniert

- einen dritten Container mit einem Mittel (III), wobei das Mittel (III) enthält:

  (a4) mindestens einen nichtionischen Emulgator der Formel (E-II) und/oder
  (a5) mindestens einen nichtionischen Emulgator der Formel (E-III),

wobei die Inhaltsstoffe (a4) und (a5) bei der Beschreibung des ersten Erfindungsgegenstands bereits im Detail offenbart wurden.

Ein weiterer Gegenstand der vorliegenden Anmeldung ist eine Mehrkomponenten-Verpackungseinheit (Kit-of-parts) zum Färben von keraitnischem Material, inbesondere menschlichen Haaren, umfassend getrennt konfektioniert

- einen ersten Container mit einem Mittel (I), wobei das Mittel (I) enthält:
  (a1) mindestens einen nichtionischen Emulgator mit einem HLB-Wert von 4,0 bis 12,0,
- einen zweiten Container mit einem Mittel (II), wobei das Mittel (II) enthält:
  (a2) mindestens Pigment,
- einen dritten Container mit einem Mittel (III); wobei das Mittel (III) enthält:
  (a3) mindestens ein aminofunktionalisiertes Silikonpolymer,

wobei die Inhaltsstoffe (a1), (a2) und (a3) bei der Beschreibung des ersten Erfindungsgegenstands bereits im Detail offenbart wurden.

Betreffend die weiteren bevorzugten Ausführungsformen der erfindungsgemäßen Verfahren und der erfindungsgemäßen Mehrkomponenten-Verpackungseinheit gilt *mutatis mutantis* das zum erfindungsgemäßen Mittel gesagte.

Beispiele

1. Formulierungen

Es wurden die folgenden Formulierungen hergestellt (alle Angaben, sofern nichts anderes angegeben ist, in Gewichtsprozent):

| anwendungsbereites Färbemittel | V | E |
|---|---|---|
| Cetylalkohol | 3,6 | 3,6 |
| Stearylalkohol | 2,0 | 2,0 |
| Paraffinum Liquidum | 2,1 | 2,1 |
| Ceteareth-30 | 1,2 | 1,2 |
| Ceteareth-100 | 0,6 | 0,6 |
| Glycerylstearat | 0,6 | 0,6 |
| Kaliumdihydrogenphosphat | 0,35 | 0,35 |
| Dinatriumhydrogenphosphat | 0,72 | 0,72 |
| Phenoxyethanol | 0,9 | 0,9 |
| Laureth-2 (Arlypon F, BASF) | --- | 1,0 |
| Pigment Blue 60 (Goldmann, CI 74160) (a2) | 1,0 | 1,0 |
| Aminosilikon (Dow Corning 2-8566 (Siloxanes and Silicones, 3-[(2-Aminoethyl)amino]-2-methylpropyl Me, Di-Me-Siloxane) (a3) | 1,0 | 1,0 |
| Wasser | ad 100 | ad 100 |

2. Herstellung der anwendungsbereiten Mittel und Anwendung

Zunächst wurde eine Träger-Formulierung bzw eine Basis-Creme hergestellt. Zu diesem Zweck wurden Cetylalkohol, Stearylalkohol und Paraffinum Liquidum vermischt und unter Rühren auf 80 °C erhitzt. Dann wurden Ceteareth-30, Ceteareth-100 und Glycerylstearat unter Rühren hinzugegeben. Mit Wasser wurde jeweils auf die entsprechende Menge aufgefüllt, dann wurden die Phosphatsalze und Phenoxyethanol hinzugefügt. Diese Basis-Creme wurde dann entsprechend den Angaben in der Tabelle mit den Inhaltstoffen (a1), (a2) und (a3) versetzt.

| V | E1 |
|---|---|
| 98,0 g Basiscreme wurden vorgelegt. Unter Rühren wurde die Creme mit 1,0 g Pigment (a2) und 1,0 g Aminosilikon (a3) vermischt. | 97,0 g Basiscreme wurden vorgelegt. 1,0 g Pigment (a2) wurden in 1,0 g Laureth-2 (a1) vordispergiert. Unter Rühren wurde die Creme mit der Vordispersion und dann mit 1,0 g Aminosilikon (a3) vermischt. |

Die auf diese Weise erhaltenen anwendungsbereiten Mittel wurde auf eine Haarsträhne (Firma Kerling, Typ "Euronatur-haar weiß" (ENH)) aufgetragen (Flottenverhältnis: 1 g Mittel pro g Haarsträhne) und für drei Minuten einwirken gelassen. Im Anschluss daran wurden die Haarsträhne gründlich (1 Minute) mit Wasser ausgewaschen und getrocknet.

3. Messung der Farbintensität

Vor dem unter Punkt 2 beschriebenen Färbevorgang wurden die Haarsträhnen mit einem Farbmessgerät der Firma Datacolor, Typ Spectraflash 450 vermessen. Nach dem Färben und Trocknen wurden die Strähnen erneut farbmetrisch vermessen.

Der für die Beurteilung der Farbintensität herangezogene dL-Wert ergibt sich aus den an der jeweiligen Strähne gemessenen Farbmesswerten wie folgt:

$$dL = [L_0 - Li]$$

$L_0$, = Messwert vor der Färbung
$L_i$ = Messwert nach der Färbung

Je größer der dL-Wert ist, desto dunkler ist die gefärbte Strähne im Vergleich zur ungefärbten Strähne und desto höher ist die Farbintensität.

| Farbintensitäten | | L | a | b | dL |
|---|---|---|---|---|---|
| V | vor dem Färben | 71,18 | 3,90 | 28,44 | 42,95 |
| | nach dem Färben | 28,23 | 3,41 | -23,61 | |
| E | vor dem Färben | 71,34 | 3,70 | 27,43 | 53,16 |
| | nach dem Färben | 18,18 | 6,66 | -16,04 | |

## Patentansprüche

1. Mittel zum Färben von keratinischem Material, insbesondere menschlichen Haaren, enthaltend

    (a1) mindestens einen nichtionischen Emulgator mit einem HLB-Wert von 4,0 bis 12,0, in einer Gesamtmenge von 0,2 bis 8,0 Gew.-%, bezogen auf das Gesamtgewicht des Mittels
    (a2) mindestens ein Pigment, und
    (a3) mindestens ein aminofunktionalisiertes Silikonpolymer,
    wobei es mindestens einen nichtionischen Emulgator (a1) mit einem HLB-Wert von 4,5 bis 10,0 enthält.

2. Mittel nach Anspruch 1, **dadurch gekennzeichnet, dass** es (a1) mindestens einen nichtionischen Emulgator mit einem HLB-Wert von 5,5 bis 9,5, und besonders bevorzugt von 6,0 bis 9,0 enthält.

3. Mittel nach einem der Ansprüche 1 bis 2, **dadurch gekennzeichnet, dass** es (a1) mindestens einen nichtionischen Emulgator enthält, der ausgewählt ist aus der Gruppe aus Laureth-2, Laureth-3, Laureth-4, Steareth-2, Steareth-3, Steareth-4, Steareth-5, Steareth-6, Ceteth-2, Ceteth-3, Ceteth-4, Ceteth-5 Ceteareth-2, Ceteareth-3, Ceteareth-4, Ceteareth-5, Beheneth-5, Cocamide MEA, Cocamide MIPA, Cocamide DEA, Stearamide DEA, Myristamide DEA, Myristamide MEA, Polyglyceryl-2 distearate, Polyglyceryl-3 distearate, Polyglyceryl-2 stearate, Polyglyceryl-3 stearate, Sorbitan distearate, Sorbitan palmitate, Sorbitan stearate, Myristyl glucoside, Cetearyl glucoside und Arachidyl glucoside.

4. Mittel nach einem der Ansprüche 1 bis 3, **dadurch gekennzeichnet, dass** es mindestens einen nichtionischen Emulgator (a1) der Formel (E-I) enthält,

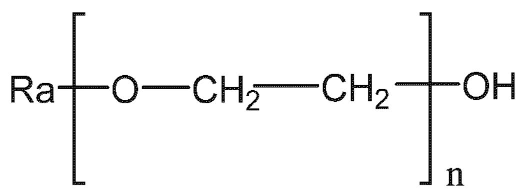

(E-I)

worin

    Ra für eine gesättigte oder ungesättigte, unverzweigte oder verzweigte $C_{12}$-$C_{24}$-Alkylgruppe steht und
    n für eine ganze Zahl von 1 bis 6, bevorzugt für eine ganze Zahl von 1 bis 5, und besonders bevorzugt für eine ganze Zahl von 2 bis 4 steht.

5. Mittel nach einem der Ansprüche 1 bis 4, **dadurch gekennzeichnet, dass** es - bezogen auf das Gesamtgewicht des Mittels - einen oder mehrere nichtionische Emulgatoren (a1) in einer Gesamtmenge von von 0,4 bis 6,0 Gew.-% und ganz besonders bevorzugt von 0,8 bis 2,5 Gew.-% enthält.

6. Mittel nach einem der Ansprüche 1 bis 5, **dadurch gekennzeichnet, dass** es mindestens ein anorganisches Pigment (a2) enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

7. Mittel nach einem der Ansprüche 1 bis 6, **dadurch gekennzeichnet, dass** es mindestens ein organisches Pigment (a2) enthält, das bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

8. Mittel nach einem der Ansprüche 1 bis 7, **dadurch gekennzeichnet, dass** es - bezogen auf das Gesamtgewicht des Mittels (a) - ein oder mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

9. Mittel nach einem der Ansprüche 1 bis 8, **dadurch gekennzeichnet, dass** es mindestens ein aminofunktionalisiertes Silikonpolymer (a3) mit mindestens einer sekundären Aminogruppe enthält.

10. Mittel nach einem der Ansprüche 1 bis 9, **dadurch gekennzeichnet, dass** es mindestens ein aminofunktionalisiertes Silikonpolymer (a3) enthält, das mindestens eine Struktureinheit der Formel (Si-Amino) umfasst,

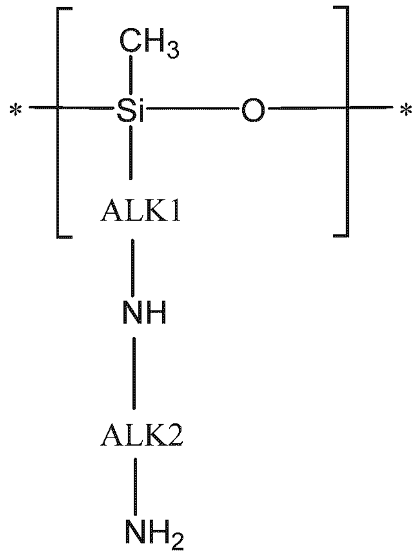

(Si-Amino)

wobei

ALK1 und ALK2 unabhängig voneinander für eine lineare oder verzweigte, zweiwertige $C_1$-$C_{20}$-Alkylengruppe stehen.

11. Mittel nach einem der Ansprüche 1 bis 10, **dadurch gekennzeichnet, dass** es mindestens ein aminofunktionalisiertes Silikonpolymer (a3) enthält, das Struktureinheiten der Formel (Si-I) und der Formel (Si-II) umfasst

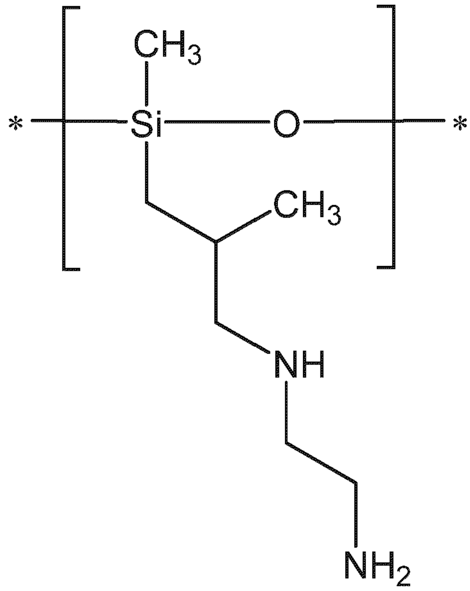

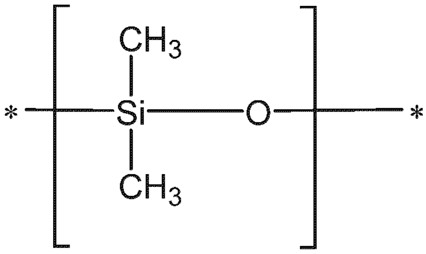

(Si-I)

(Si-II).

12. Mittel nach einem der Ansprüche 1 bis 11, **dadurch gekennzeichnet, dass** es - bezogen auf das Gesamtgewicht des Mittels - ein oder mehrere aminofunktionalisierte Silikonpolymere (a3) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

13. Mittel nach einem der Ansprüche 1 bis 12, **dadurch gekennzeichnet, dass** es zusätzlich mindestens einen weiteren nichtionischen Emulgator (a4) der Formel (E-II) enthält,

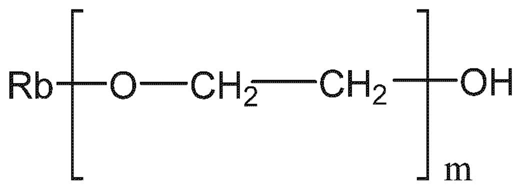

(E-II)

worin

Rb für eine gesättigte oder ungesättigte, unverzweigte oder verzweigte $C_{12}$-$C_{24}$-Alkylgruppe steht und und m für eine ganze Zahl von 20 bis 40, bevorzugt für eine ganze Zahl von 20 bis 35 und besonders bevorzugt für die Zahl 30, steht.

14. Mittel nach einem der Ansprüche 1 bis 13, **dadurch gekennzeichnet, dass** es zusätzlich mindestens einen weiteren nichtionischen Emulgator (a5) der Formel (E-III) enthält,

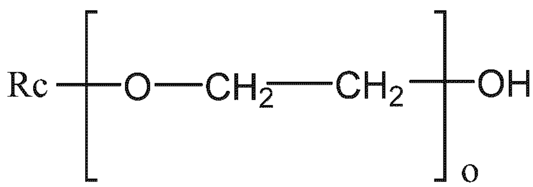

(E-III)

worin

Rc für eine gesättigte oder ungesättigte, unverzweigte oder verzweigte $C_{12}$-$C_{24}$-Alkylgruppe steht und und o für eine ganze Zahl von 80 bis 120, bevorzugt für eine ganze Zahl von 90 bis 110 und besonders bevorzugt für die Zahl 100, steht.

**15.** Verfahren zum Färben von keratinischem Material, insbesondere menschlichen Haaren, bei welchem ein Mittel, wie es in den Ansprüchen 1 bis 14 beschrieben ist, auf die keratinischen Fasern aufgetragen wird und gegebenenfalls nach einer Einwirkzeit von 30 Sekunden bis 45 Minuten wieder ausgespült wird.

**16.** Verfahren zum Färben von keratinischem Material, insbesondere menschlichen Haaren, umfassend die folgenden Schritte:

(1) Bereitstellung eines Mittels (I), wobei das Mittel (I) enthält:

(a1) mindestens einen nichtionischen Emulgator mit einem HLB-Wert von 4,0 bis 12,0, und
(a2) mindestens ein Pigment,

(2) Bereitstellung eines Mittels (II), wobei das Mittel (II) enthält:
(a3) mindestens ein aminofunktionalisiertes Silikonpolymer, und
(3) gegebenenfalls Bereitstellung eines Mittels (III), wobei das Mittel (III) enhält:

(a4) mindestens einen nichtionischen Emulgator (a4) der Formel (E-II) und/oder
(a5) mindestens einen nichtionischen Emulgator (a5) der Formel (E-III),

(4) Herstellung einer Anwendungsmischung durch Vermischen des Mittels (I) mit dem Mittel (II) und gegebenenfalls mit dem Mittel (III),
(5) Applizieren der in Schritt (4) hergestellten Anwendungsmischung auf dem keratinischen Material,
(6) Einwirken der in Schritt (5) applizierten Anwendungsmischung auf dem keratinischen Material und
(7) Ausspülen der Anwendungsmischung mit Wasser,

wobei die Inhaltsstoffe (a1), (a2), (a3), (a4) und (a5) in den Ansprüchen 1 bis 14 definiert sind.

**17.** Mehrkomponenten-Verpackungseinheit (Kit-of-parts) zum Färben von keratinischem Material, umfassend getrennt voneinander konfektioniert

- einen ersten Container mit einem Mittel (I), wobei das Mittel (I) enthält:

(a1) mindestens einen nichtionischen Emulgator mit einem HLB-Wert von 4,0 bis 12,0,
(a2) mindestens ein Pigment, und

- einen zweiten Container mit einem Mittel (II), wobei das Mittel (II) enthält:

(a3) mindestens ein aminofunktionalisiertes Silikonpolymer,

wobei die Inhaltsstoffe (a1), (a2) und (a3) in den Ansprüchen 1 bis 14 definiert sind.

**18.** Mehrkomponenten-Verpackungseinheit (Kit-of-parts) nach Anspruch 17, umfassend getrennt konfektioniert

- einen dritten Container mit einem Mittel (III), wobei das Mittel (III) enthält:

(a4) mindestens einen nichtionischen Emulgator der Formel (E-II) und/oder
(a5) mindestens einen nichtionischen Emulgator der Formel (E-III),

wobei die Inhaltsstoffe (a4) und (a5) in den Ansprüchen 13 bis 14 definiert sind.

## Claims

**1.** An agent for dyeing keratinous material, in particular human hair, containing

(a1) at least one non-ionic emulsifier having an HLB value from 4.0 to 12.0, in a total amount from 0.2 to 8.0 wt.% based on the total weight of the agent,
(a2) at least one pigment, and

(a3) at least one amino-functionalized silicone polymer,

wherein it contains at least one non-ionic emulsifier (a1) having an HLB value from 4.5 to 10.0.

2. The agent according to claim 1, **characterized in that** it contains
(a1) at least one non-ionic emulsifier having an HLB value from 5.5 to 9.5, and particularly preferably from 6.0 to 9.0.

3. The agent according to one of claims 1 to 2, **characterized in that** it contains
(a1) at least one non-ionic emulsifier selected from the group consisting of laureth-2, laureth-3, laureth-4, steareth-2, steareth-3, steareth-4, steareth-5, steareth-6, ceteth-2, ceteth-3, ceteth-4, ceteth-5, ceteareth-2, ceteareth-3, ceteareth-4, ceteareth-5, beheneth-5, cocamide MEA, cocamide MIPA, cocamide DEA, stearamide DEA, myristamide DEA, myristamide MEA, polyglyceryl-2 distearate, polyglyceryl-3 distearate, polyglyceryl-2 stearate, polyglyceryl-3 stearate, sorbitan distearate, sorbitan palmitate, sorbitan stearate, myristyl glucoside, cetearyl glucoside and arachidyl glucoside.

4. The agent according to one of claims 1 to 3, **characterized in that** it contains at least one non-ionic emulsifier (a1) of formula (E-I),

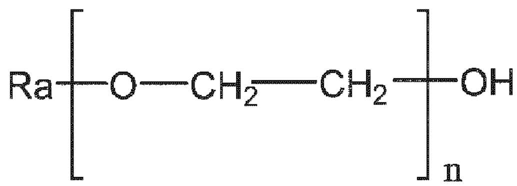

(E-I)

where

Ra represents a saturated or unsaturated, unbranched or branched $C_{12}$-$C_{24}$ alkyl group, and
n represents an integer from 1 to 6, preferably an integer from 1 to 5 and particularly preferably an integer from 2 to 4.

5. The agent according to one of claims 1 to 4, **characterized in that** it contains, based on the total weight of the agent, one or more non-ionic emulsifiers (a1) in a total amount from 0.4 to 6.0 wt.% and very particularly preferably from 0.8 to 2.5 wt.%.

6. The agent according to one of claims 1 to 5, **characterized in that** it contains at least one inorganic pigment (a2), which is preferably selected from the group consisting of colored metal oxides, metal hydroxides, metal oxide hydrates, silicates, metal sulfides, complex metal cyanides, metal sulfates, bronze pigments and/or from mica-based colored pigments which are coated with at least one metal oxide and/or a metal oxychloride.

7. The agent according to one of claims 1 to 6, **characterized in that** it contains at least one organic pigment (a2), which is preferably selected from the group consisting of carmine, quinacridone, phthalocyanine, sorghum, blue pigments having the Color Index Numbers CI 42090, CI 69800, CI 69825, CI 73000, CI 74100, CI 74160, yellow pigments having the Color Index Numbers CI 11680, CI 11710, CI 15985, CI 19140, CI 20040, CI 21100, CI 21108, CI 47000, CI 47005, green pigments having the Color Index Numbers CI 61565, CI 61570, CI 74260, orange pigments having the Color Index Numbers CI 11725, CI 15510, CI 45370, CI 71105, red pigments having the Color Index Numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

8. The agent according to one of claims 1 to 7, **characterized in that** it contains, based on the total weight of the agent (a), one or more pigments (a2) in a total amount from 0.01 to 10.0 wt.%, preferably from 0.1 to 5.0 wt.%, more preferably from 0.2 to 2.5 wt.%, and very particularly preferably from 0.25 to 1.5 wt.%.

9. The agent according to one of claims 1 to 8, **characterized in that** it contains at least one amino-functionalized silicone polymer (a3) having at least one secondary amino group.

10. The agent according to one of claims 1 to 9, **characterized in that** it contains at least one amino-functionalized

silicone polymer (a3) which comprises at least one structural unit of formula (Si-amino),

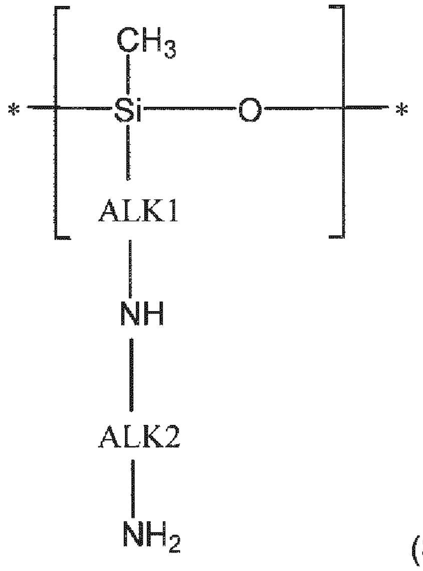

(Si-amino)

where
ALK1 and ALK2 represent, independently of one another, a linear or branched, divalent $C_1$-$C_{20}$ alkylene group.

11. The agent according to one of claims 1 to 10, **characterized in that** it contains at least one amino-functionalized silicone polymer (a3) which comprises structural units of formula (Si-I) and formula (Si-II)

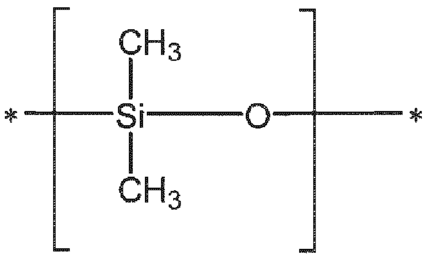

(Si-I)

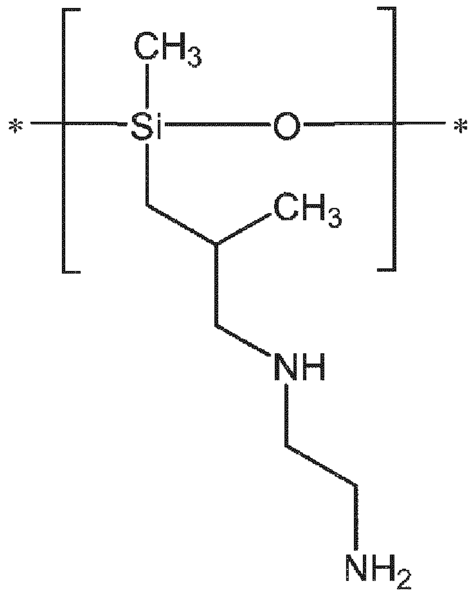

(Si-II).

12. The agent according to one of claims 1 to 11, **characterized in that** it contains, based on the total weight of the agent, one or more amino-functionalized silicone polymers (a3) in a total amount from 0.1 to 8.0 wt.%, preferably from 0.2 to 5.0 wt.%, more preferably from 0.3 to 3.0 wt.%, and very particularly preferably from 0.4 to 2.5 wt.%.

13. The agent according to one of claims 1 to 12, **characterized in that** it additionally contains at least one further non-ionic emulsifier (a4) of formula (E-II),

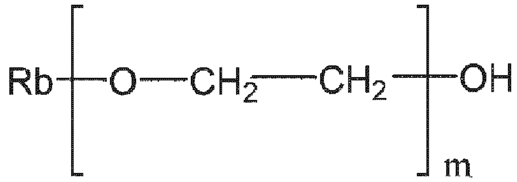

(E-II)

where

Rb represents a saturated or unsaturated, unbranched or branched $C_{12}$-$C_{24}$ alkyl group, and
m represents an integer from 20 to 40, preferably an integer from 20 to 35 and particularly preferably the integer 30.

**14.** The agent according to one of claims 1 to 13, **characterized in that** it additionally contains at least one further non-ionic emulsifier (a5) of formula (E-III),

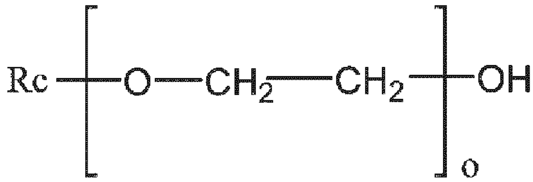

(E-III)

where

Rc represents a saturated or unsaturated, unbranched or branched $C_{12}$-$C_{24}$ alkyl group, and
O represents an integer from 80 to 120, preferably an integer from 90 to 110 and particularly preferably the integer 100.

**15.** A method for dyeing keratinous material, in particular human hair, in which an agent as described in claims 1 to 14 is applied to the keratinous fibers and optionally rinsed after an exposure time of 30 seconds to 45 minutes.

**16.** The method for dyeing keratinous material, in particular human hair, comprising the following steps:

(1) providing an agent (I), wherein the agent (I) contains:

(a1) at least one non-ionic emulsifier having an HLB value from 4.0 to 12.0, and
(a2) at least one pigment,

(2) providing an agent (II), wherein the agent (II) contains:
(a3) at least one amino-functionalized silicone polymer, and
(3) optionally providing an agent (III), wherein the agent (III) contains:

(a4) at least one non-ionic emulsifier (a4) of formula (E-II), and/or
(a5) at least one non-ionic emulsifier (a5) of formula (E-III),

(4) preparing an application mixture by mixing the agent (I) with the agent (II) and optionally with the agent (III),
(5) applying the application mixture prepared in step (4) to the keratinous material,
(6) exposing the keratinous material to the application mixture applied in step (5), and
(7) rinsing the application mixture using water,
wherein the ingredients (a1), (a2), (a3) (a4) and (a5) are defined in claims 1 to 14.

**17.** A multi-component packaging unit (kit-of-parts) for dyeing keratinous material, comprising, packaged separately from one another,

- a first container having an agent (I), wherein the agent (I) contains:

(a1) at least one non-ionic emulsifier having an HLB value from 4.0 to 12.0,
(a2) at least one pigment, and

- a second container having an agent (II), wherein the agent (II) contains:

(a3) at least one amino-functionalized silicone polymer,

wherein the ingredients (a1), (a2) and (a3) are defined in claims 1 to 14.

**18.** The multi-component packaging unit (kit-of-parts) according to claim 17, comprising, packaged separately,

- a third container having an agent (III), wherein the agent (III) contains:

(a4) at least one non-ionic emulsifier of formula (E-II), and/or
(a5) at least one non-ionic emulsifier of formula (E-III),

wherein the ingredients (a4) and (a5) are defined in claims 13 to 14.

## Revendications

**1.** Agent permettant la coloration d'une matière kératinique, en particulier de cheveux humains, contenant

(a1) au moins un émulsifiant non ionique comportant une valeur HLB allant de 4,0 à 12,0, en une quantité totale allant de 0,2 à 8,0 % en poids, par rapport au poids total de l'agent
(a2) au moins un pigment, et
(a3) au moins un polymère de silicone aminofonctionnalisé,

dans lequel il contient au moins un émulsifiant non ionique (a1) comportant une valeur HLB allant de 4,5 à 10,0.

**2.** Agent selon la revendication 1, **caractérisé en ce qu'**il
(a1) contient au moins un émulsifiant non ionique comportant une valeur HLB allant de 5,5 à 9,5, et de manière particulièrement préférée de 6,0 à 9,0.

**3.** Agent selon l'une des revendications 1 à 2, **caractérisé en ce qu'**il
(a1) contient au moins un émulsifiant non ionique qui est choisi dans le groupe constitué de laureth-2, laureth-3, laureth-4, stéareth-2, stéareth-3, stéareth-4, stéareth-5, stéareth-6, céteth-2, céteth-3, céteth-4, céteth-5, cétéareth-2, cétéareth-3, cétéareth-4, cétéareth-5, béhéneth-5, cocamide MEA, cocamide MIPA, cocamide DEA, stéaramide DEA, myristamide DEA, myristamide MEA, distéarate de polyglycéryl-2, distéarate de polyglycéryl-3, stéarate de polyglycéryl-2, stéarate de polyglycéryl-3, distéarate de sorbitan, palmitate de sorbitan, stéarate de sorbitan, glucoside de myristyle, glucoside de cétéaryle et glucoside d'arachidyle.

**4.** Agent selon l'une des revendications 1 à 3, **caractérisé en ce qu'**il contient au moins un émulsifiant non ionique (a1) de formule (E-I),

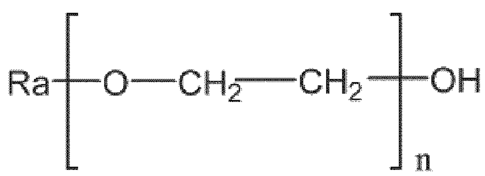

(E-I)

où

Ra représente un groupe alkyle en $C_{12}$-$C_{24}$, saturé ou insaturé, linéaire ou ramifié, et
n représente un nombre entier allant de 1 à 6, de préférence un nombre entier allant de 1 à 5, et de manière particulièrement préférée un nombre entier allant de 2 à 4.

5. Agent selon l'une des revendications 1 à 4, **caractérisé en ce qu'**il contient, par rapport au poids total de l'agent, un ou plusieurs émulsifiants non ioniques (a1) en une quantité totale allant de 0,4 à 6,0 % en poids et de manière très particulièrement préférée de 0,8 à 2,5 % en poids.

6. Agent selon l'une des revendications 1 à 5, **caractérisé en ce qu'**il contient au moins un pigment (a2) inorganique qui est de préférence choisi dans le groupe constitué d'oxydes métalliques colorés, hydroxydes métalliques colorés, oxydes métalliques hydratés colorés, silicates colorés, sulfures métalliques colorés, cyanures métalliques complexes colorés, sulfates métalliques colorés, pigments de bronze colorés et/ou de pigments colorés à base de mica qui sont recouverts d'au moins un oxyde métallique et/ou d'un oxychlorure métallique.

7. Agent selon l'une des revendications 1 à 6, **caractérisé en ce qu'**il contient au moins un pigment (a2) organique qui est de préférence choisi dans le groupe constitué de carmin, quinacridone, phtalocyanine, sorgho, pigments bleus comportant les numéros d'indice de couleur CI 42090, CI 69800, CI 69825, CI 73000, CI 74100, CI 74160, pigments jaunes comportant les numéros d'indice de couleur CI 11680, CI 11710, CI 15985, CI 19140, CI 20040, CI 21100, CI 21108, CI 47000, CI 47005, pigments verts comportant les numéros d'indice de couleur CI 61565, CI 61570, CI 74260, pigments orange comportant les numéros d'indice de couleur CI 11725, CI 15510, CI 45370, CI 71105, pigments rouges comportant les numéros d'indice de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

8. Agent selon l'une des revendications 1 à 7, **caractérisé en ce qu'**il contient, par rapport au poids total de l'agent (a), un ou plusieurs pigments (a2) en une quantité totale allant de 0,01 à 10,0 % en poids, de préférence de 0,1 à 5,0 % en poids, plus préférablement de 0,2 à 2,5 % en poids et de manière très particulièrement préférée de 0,25 à 1,5 % en poids.

9. Agent selon l'une des revendications 1 à 8, **caractérisé en ce qu'**il contient au moins un polymère de silicone aminofonctionnalisé (a3) comportant au moins un groupe amine secondaire.

10. Agent selon l'une des revendications 1 à 9, **caractérisé en ce qu'**il contient au moins un polymère de silicone aminofonctionnalisé (a3) qui comprend au moins un motif structural de formule (Si-Amino),

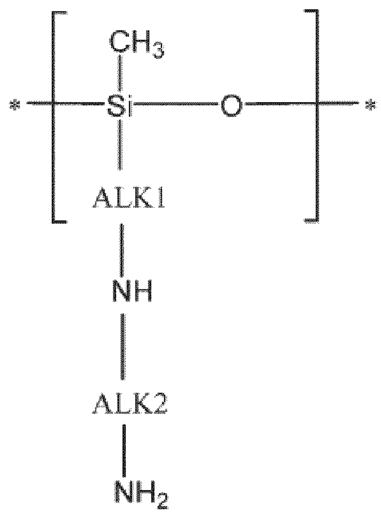

(Si-Amino)

où
ALK1 et ALK2 représentent, indépendamment l'un de l'autre, un groupe alkylène en $C_1$-$C_{20}$ bivalent, linéaire ou ramifié.

11. Agent selon l'une des revendications 1 à 10, **caractérisé en ce qu'**il contient au moins un polymère de silicone aminofonctionnalisé (a3) qui comprend des motifs structuraux de formule (Si-I) et de formule (Si-II)

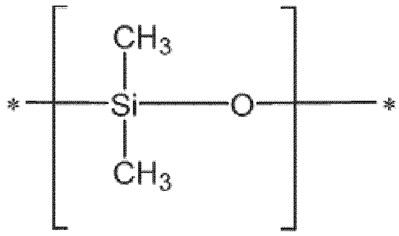

(Si-I)

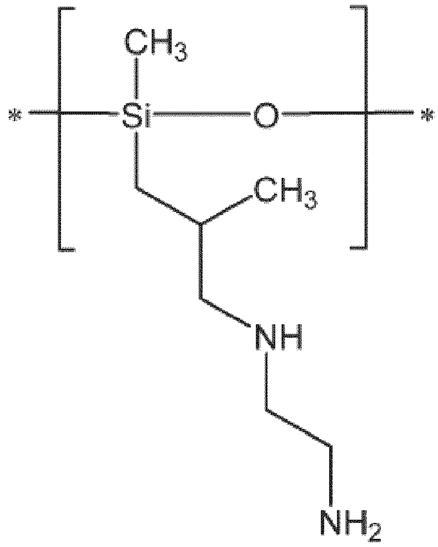

(Si-II).

**12.** Agent selon l'une des revendications 1 à 11, **caractérisé en ce qu'**il contient, par rapport au poids total de l'agent, un ou plusieurs polymères de silicone aminofonctionnalisés (a3) en une quantité totale allant de 0,1 à 8,0 % en poids, de préférence de 0,2 à 5,0 % en poids, plus préférablement de 0,3 à 3,0 % en poids et de manière très particulièrement préférée de 0,4 à 2,5 % en poids.

**13.** Agent selon l'une des revendications 1 à 12, **caractérisé en ce qu'**il contient en outre au moins un autre émulsifiant non ionique (a4) de formule (E-II),

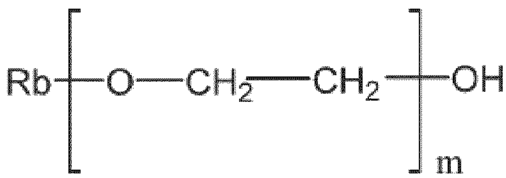

(E-II)

où

Rb représente un groupe alkyle en $C_{12}$-$C_{24}$, saturé ou insaturé, linéaire ou ramifié, et
m représente un nombre entier allant de 20 à 40, de préférence un nombre entier allant de 20 à 35, et de manière particulièrement préférée le nombre 30.

**14.** Agent selon l'une des revendications 1 à 13, **caractérisé en ce qu'il** contient en outre au moins un autre émulsifiant non ionique (a5) de formule (E-III),

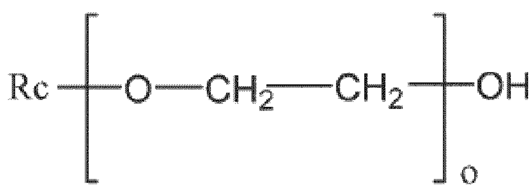

(E-III)

où

Rc représente un groupe alkyle en $C_{12}$-$C_{24}$, saturé ou insaturé, linéaire ou ramifié, et
o représente un nombre entier allant de 80 à 120, de préférence un nombre entier allant de 90 à 110, et de manière particulièrement préférée le nombre 100.

**15.** Procédé permettant la coloration d'une matière kératinique, en particulier de cheveux humains, dans lequel un agent

tel que décrit dans les revendications 1 à 14 est appliqué sur les fibres kératiniques et est éventuellement rincé après un temps d'action allant de 30 secondes à 45 minutes.

**16.** Procédé permettant la coloration d'une matière kératinique, en particulier de cheveux humains, comprenant les étapes suivantes :

(1) fourniture d'un agent (I), dans lequel l'agent (I) contient :

(a1) au moins un émulsifiant non ionique comportant une valeur HLB allant de 4,0 à 12,0, et
(a2) au moins un pigment,

(2) fourniture d'un agent (II), dans lequel l'agent (II) contient :
(a3) au moins un polymère de silicone aminofonctionnalisé, et
(3) fourniture éventuelle d'un agent (III), dans lequel l'agent (III) contient :

(a4) au moins un émulsifiant non ionique (a4) de formule (E-II), et/ou
(a5) au moins un émulsifiant non ionique (a5) de formule (E-III),

(4) préparation d'un mélange à appliquer en mélangeant l'agent (I) avec l'agent (II) et éventuellement avec l'agent (III),
(5) application du mélange à appliquer préparé à l'étape (4) sur la matière kératinique,
(6) action du mélange à appliquer appliqué à l'étape (5) sur la matière kératinique et
(7) rinçage du mélange à appliquer avec de l'eau,

dans lequel les ingrédients (a1), (a2), (a3), (a4) et (a5) sont définis dans les revendications 1 à 14.

**17.** Unité d'emballage à plusieurs composants (kit de pièces) permettant la coloration d'une matière kératinique, comprenant, conditionnés de manière à être séparés les uns des autres,

- un premier récipient comportant un agent (I), dans laquelle l'agent (I) contient :

(a1) au moins un émulsifiant non ionique comportant une valeur HLB allant de 4,0 à 12,0,
(a2) au moins un pigment, et

- un deuxième récipient comportant un agent (II), dans laquelle l'agent (II) contient :
(a3) au moins un polymère de silicone aminofonctionnalisé,

dans laquelle les ingrédients (a1), (a2) et (a3) sont définis dans les revendications 1 à 14.

**18.** Unité d'emballage à plusieurs composants (kit de pièces) selon la revendication 17, comprenant, conditionné de manière à être séparé,

- un troisième récipient comportant un agent (III), dans laquelle l'agent (III) contient :

(a4) au moins un émulsifiant non ionique de formule (E-II), et/ou
(a5) au moins un émulsifiant non ionique de formule (E-III),

dans laquelle les ingrédients (a4) et (a5) sont définis dans les revendications 13 à 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102018222022 A1
- DE 19738866 A


### In der Beschreibung aufgeführte Nicht-Patentliteratur


- Die Körperpflegemittel. **HUGO JANISTYN**. Handbuch der Kosmetika und Riechstoffe. Dr. Alfred Hüthig Verlag, 1973, vol. III, 68-78
- **HUGO JANISTYN**. Taschenbuch der modernen Parfümerie und Kosmetik. Wissenschaftliche Verlagsgesellschaft m.b.H., 1974, 466-474
- *Publikation J. Soc. Cosm. Chem.*, 1954, vol. 5, 249-256
- *CHEMICAL ABSTRACTS*, 68439-50-9
- *CHEMICAL ABSTRACTS*, 106842-44-8
- *CHEMICAL ABSTRACTS*, 68439-49-6
- *CHEMICAL ABSTRACTS*, 9005-00-9
- *CHEMICAL ABSTRACTS*, 57-55-6
- *CHEMICAL ABSTRACTS*, 4254-14-2
- *CHEMICAL ABSTRACTS*, 4254-153
- *CHEMICAL ABSTRACTS*, 107-21-1
- *CHEMICAL ABSTRACTS*, 56-81-5
- *CHEMICAL ABSTRACTS*, 122-99-6
- *CHEMICAL ABSTRACTS*, 7778-77-0
- *CHEMICAL ABSTRACTS*, 7558-80-7
- *CHEMICAL ABSTRACTS*, 10049-21-5
- *CHEMICAL ABSTRACTS*, 13472-35-0
- *CHEMICAL ABSTRACTS*, 7758-11-4
- *CHEMICAL ABSTRACTS*, 16788-57-1
- *CHEMICAL ABSTRACTS*, 7558-79-4
- *CHEMICAL ABSTRACTS*, 10028-24-7
- *CHEMICAL ABSTRACTS*, 7782-85-6
- *CHEMICAL ABSTRACTS*, 10039-32-4